# EUROPEAN PATENT APPLICATION

(11) **EP 4 835 046 A1**
(43) Date of publication of application: **04.11.2026**
(21) Application number: 24910724.4
(22) Date of filing: 11.12.2024
(51) Int. Cl.: C12M 1/34, C12M 1/00, C12M 1/36, C12M 1/38, C12M 1/42, C12Q 1/68

(54) **SAMPLE ANALYZER**

(30) Priority: 29.12.2023 CN 202311868300; 29.04.2024 CN 202410538392
(71) Applicant: Shenzhen Mindray Bio-Medical Electronics Co., Ltd., Shenzhen, Guangdong 518057 (CN)
(72) Inventor: JIANG, Bin, Shenzhen, Guangdong 518057 (CN); LIU, Jin, Shenzhen, Guangdong 518057 (CN); CHEN, Jun, Shenzhen, Guangdong 518057 (CN); GAO, Zaixing, Shenzhen, Guangdong 518057 (CN)
(74) Representative: KIPA AB
(86) International application number: PCT/CN2024/138488
(87) International publication number: WO 2025/139793

(57) **Abstract**

The present application is suitable for the field of in-vitro diagnosis apparatuses. Provided is a sample analyzer, the sample analyzer comprising a sample distribution device, a nucleic acid extraction device, a pipetting device, an amplification device and a test device. The nucleic acid extraction device comprises a first functional assembly, a second functional assembly and a transfer assembly, the first functional assembly being provided above or below the second functional assembly, the transfer assembly being used for transferring a nucleic acid extraction container between the first functional assembly and the second functional assembly, the first functional assembly being used for at least performing magnetic attraction and liquid suction actions on a liquid in the nucleic acid extraction container, and the second functional assembly being at least used for incubating the liquid in the nucleic acid extraction container. The first functional assembly and the second functional assembly which are used for bearing the nucleic acid extraction container to perform the functional actions in the nucleic acid extraction device being arranged up and down can reduce the space occupied by the nucleic acid extraction device in the horizontal direction, thereby facilitating miniaturization of the sample analyzer.

## Description

### CROSS-REFERENCE TO RELATED APPLICATIONS

The disclosure is filed based on and claims priority to Chinese Patent Application No. 2023118683007, filed on December 29, 2023, and Chinese Patent Application No. 2024105383920, filed on April 29, 2024, which are incorporated herein by reference in their entirety.

### TECHNICAL FIELD

The disclosure relates to the field of in-vitro diagnostic apparatuses, and in particular, to a sample analyzer.

### BACKGROUND

Molecular diagnostic technology refers to a technology that uses DNA and RNA as diagnostic materials and employs molecular biology technology to detect the presence, defects, or abnormal expression of genes, so as to diagnose human physiological conditions and diseases. The basic principle thereof is to detect whether there are changes in the structure of DNA or RNA, variations in quantity, or abnormalities in expression functions, so as to determine whether the subject suffers from abnormal changes at the genetic level, which is of great significance for the prevention, prediction, diagnosis, treatment, and prognosis of diseases. A molecular diagnostic integrated machine is a test apparatus for implementing molecular diagnostic testing. Since molecular diagnosis involves a plurality of operational steps, such as sample preparation, nucleic acid extraction, and reaction system construction, the corresponding molecular diagnostic integrated machine is provided with a large number of functional modules. Moreover, to achieve a complete sample-to-result process, numerous auxiliary functional modules for reagent management, waste management, contamination prevention management, etc. are further provided. An integrated machine formed by integrating such a large number of modules often has very large overall dimensions. A molecular laboratory in which the molecular diagnostic integrated machine is placed is required to implement partitioned isolation and ensure a negative-pressure experimental environment in order to prevent contamination. Accordingly, given the generally limited total floor area of current molecular laboratories, where space is extremely valuable, the deployment of a bulky integrated machine in a molecular laboratory has become a significant challenge for users. For manufacturers of molecular diagnostic integrated machines, reducing the size of the machine has also become imperative.

In a molecular diagnostic integrated machine provided in the related art, a plurality of stations of a nucleic acid extraction device are arranged in a linear sequence in a horizontal direction. As a result, the nucleic acid extraction device has a relatively large overall size and occupies a large amount of horizontal space, seriously affecting the miniaturization design of the molecular diagnostic integrated machine. The nucleic acid extraction device is one of the functional modules occupying the largest amount of space in the molecular diagnostic integrated machine, and optimization of the design and structural layout thereof is of great significance to the compact design of the entire molecular diagnostic integrated machine.

### SUMMARY

A first object of the disclosure is to provide a sample analyzer, which is intended to solve the technical problem of a nucleic acid extraction device in the related art occupying a large amount of horizontal space.

To achieve the above object, the disclosure provides the following solution:

A sample analyzer, including:
a sample distribution device configured to aspirate a sample from a sample container and dispense at least part of the aspirated sample into a nucleic acid extraction container;
a nucleic acid extraction device configured to extract nucleic acids from a liquid containing at least the sample and a reagent in the nucleic acid extraction container to obtain a nucleic acid extract, the reagent containing magnetic beads;
a pipetting device configured to transfer the nucleic acid extract from the nucleic acid extraction container to an amplification reaction container;
an amplification device configured to amplify the nucleic acid extract in the amplification reaction container to obtain a test solution; and
a test device configured to test the test solution,
wherein the nucleic acid extraction device includes a first functional assembly, a second functional assembly, and a transfer assembly, the first functional assembly being disposed above or below the second functional assembly, and the transfer assembly being configured to transfer the nucleic acid extraction container between the first functional assembly and the second functional assembly so as to perform the nucleic acid extraction to obtain the nucleic acid extract; and the first functional assembly being configured to at least perform magnetic attraction and a liquid aspiration operation on the liquid in the nucleic acid extraction container, and the second functional assembly being configured to at least incubate the liquid in the nucleic acid extraction container.

As an implementation, the first functional assembly includes a first support member, a first pipetting member, and a first magnetic attraction member, the first support member being configured to support the nucleic acid extraction container, the first magnetic attraction member being configured to perform a first magnetic attraction on the liquid containing at least the sample and the reagent in the nucleic acid extraction container located on the first support member so as to attract the magnetic beads in the nucleic acid extraction container, and the first pipetting member being configured to perform a first liquid aspiration operation on the liquid in the nucleic acid extraction container located on the first support member and subjected to the first magnetic attraction by the first magnetic attraction member; and the transfer assembly is configured to transfer the nucleic acid extraction container between the first support member and the second functional assembly. The magnetic attraction includes the first magnetic attraction, and the liquid aspiration operation includes the first liquid aspiration operation.

As an implementation, the first functional assembly further includes a liquid dispensing member configured to perform a first liquid dispensing operation for dispensing a cleaning liquid into the nucleic acid extraction container located on the first support member.

As an implementation, the first functional assembly further includes a second support member, a second pipetting member, and a second magnetic attraction member, the second support member being configured to support the nucleic acid extraction container, the second magnetic attraction member being configured to perform a second magnetic attraction on a liquid containing at least the cleaning liquid in the nucleic acid extraction container located on the second support member, and the second pipetting member being configured to perform a second liquid aspiration operation on the liquid in the nucleic acid extraction container located on the second support member and subjected to the second magnetic attraction by the second magnetic attraction member; and the transfer assembly is further configured to transfer the nucleic acid extraction container between the second support member and the second functional assembly. The magnetic attraction further includes the second magnetic attraction, and the liquid aspiration operation further includes the second liquid aspiration operation.

As an implementation, the first functional assembly further includes a third support member and a third pipetting member, the third support member being configured to support the nucleic acid extraction container, and the third pipetting member being configured to perform a second liquid dispensing operation for dispensing an elution reagent into the nucleic acid extraction container located on the third support member. The transfer assembly is further configured to transfer the nucleic acid extraction container between the third support member and the second functional assembly.

As an implementation, the sample analyzer further includes a first lifting member. The first lifting member is connected to the second magnetic attraction member and is configured to drive the second magnetic attraction member to move up and down relative to the nucleic acid extraction container.

As an implementation, a reaction well, a first tip well, and a second tip well are formed in the nucleic acid extraction container. The reaction well is configured to receive the sample and the reagent for nucleic acid extraction, the first tip well is configured to accommodate a first pipetting tip, and the second tip well is configured to accommodate a second pipetting tip. A capacity of the first pipetting tip is greater than a capacity of the second pipetting tip.

The first pipetting member includes a first tip connecting component and a first aspiration-discharge drive component. The first tip connecting component is configured to be removably connected to the first pipetting tip so as to perform liquid aspiration by the first pipetting tip under power provided by the first aspiration-discharge drive component.

The third pipetting member includes a second tip connecting component and a second aspiration-discharge drive component, the second tip connecting component being configured to be removably connected to the second pipetting tip so as to perform liquid aspiration by the second pipetting tip under power provided by the second aspiration-discharge drive component.

As an implementation, a waste liquid well is further formed in the nucleic acid extraction container, and the first pipetting member and the second pipetting member are further configured to dispense liquid aspirated from the reaction well into the waste liquid well.

As an implementation, at least two first support members are provided, with each first support member being correspondingly provided with at least one liquid dispensing member and at least one first pipetting member.

As an implementation, the first support member, the second support member, and the third support member are disposed side-by-side in a horizontal direction.

As an implementation, the second functional assembly includes a fourth support member and a heating member, the fourth support member being configured to support the nucleic acid extraction container for performing an incubation operation, and the heating member being configured to heat the liquid containing at least the sample and the reagent in the nucleic acid extraction container located on the fourth support member. The fourth support member includes a lysis zone, a washing zone, a drying zone, and an elution zone. An accommodating hole for placement of the nucleic acid extraction container is formed in each of the lysis zone, the washing zone, the drying zone and the elution zone, and the transfer assembly is configured to transfer the nucleic acid extraction container between the lysis zone, the washing zone and the first support member, between the washing zone, the drying zone and the second support member, and between the drying zone, the elution zone and the third support member.

As an implementation, the fourth support member includes an incubation tray, and the heating member is configured to heat the nucleic acid extraction container on the incubation tray. The lysis zone, the washing zone, the drying zone, and the elution zone are formed on the incubation tray, and a first transfer station is further formed on the incubation tray. The incubation tray is configured to convey the nucleic acid extraction container located in each of the lysis zone, the washing zone, the drying zone and the elution zone to the first transfer station, and the transfer assembly is configured to convey the nucleic acid extraction container located at the first transfer station to the first functional assembly.

As an implementation, the sample analyzer further includes a controller configured to:
control the transfer assembly to convey the nucleic acid extraction container subjected to the incubation operation in the lysis zone to the first support member;
control the first pipetting member to perform the first liquid aspiration operation on the liquid in the nucleic acid extraction container in the first support member, and control the liquid dispensing member to perform the first liquid dispensing operation on the nucleic acid extraction container in the first support member;
control the transfer assembly to transfer the nucleic acid extraction container subjected to the first liquid dispensing operation to the washing zone to perform the incubation operation;
control the transfer assembly to transfer the nucleic acid extraction container subjected to the incubation operation in the washing zone to the second support member;
control the second pipetting member to perform the second liquid aspiration operation on the liquid in the nucleic acid extraction container in the second support member;
control the transfer assembly to transfer the nucleic acid extraction container subjected to the second liquid aspiration operation to the drying zone to perform a drying operation on the nucleic acid extraction container;
control the transfer assembly to transfer the nucleic acid extraction container subjected to the drying operation in the drying zone to the third support member;
control the third pipetting member to perform the second liquid dispensing operation on the nucleic acid extraction container in the third support member; and
control the transfer assembly to transfer the nucleic acid extraction container subjected to the second liquid dispensing operation to the elution zone to perform an elution operation.

As an implementation, a bottom of the accommodating hole in the elution zone is positioned higher than a bottom of the accommodating hole in the lysis zone, higher than a bottom of the accommodating hole in the washing zone, and higher than a bottom of the accommodating hole in the drying zone; and/or
the bottom of the accommodating hole in the lysis zone is positioned at the same height as the bottom of the accommodating hole in the washing zone and the bottom of the accommodating hole in the drying zone.

As an implementation, the second functional assembly includes a fourth support member, a heating member, and a mixing member. The fourth support member is configured to support the nucleic acid extraction container for performing an incubation operation, and the heating member is configured to heat the liquid containing at least the sample and the reagent in the nucleic acid extraction container located on the fourth support member. The mixing member is configured to mix the liquid containing at least the sample and the reagent in the nucleic acid extraction container located on the fourth support member.

As an implementation, the second functional assembly further includes a first driving member configured to drive the fourth support member to move such that the fourth support member drives the nucleic acid extraction container to move.

The mixing member includes a plurality of magnets. The plurality of magnets are alternately arranged on opposite sides of a movement trajectory of the nucleic acid extraction container in a horizontal direction.

As an implementation, the mixing member includes at least a first magnet array and a second magnet array extending horizontally, with each magnet array including a plurality of magnets spaced apart and mounted on a magnet bracket.

The fourth support member includes a container receiving portion for placement of the nucleic acid extraction container.

The fourth support member and the mixing member are disposed with respect to each other such that when the nucleic acid extraction container is received in the container receiving portion, at least a bottom of the nucleic acid extraction container is located between the first magnet array and the second magnet array.

The fourth support member and the mixing member are capable of moving horizontally relative to each other such that the mixing member is capable of mixing the liquid containing at least the sample and magnetic beads in the nucleic acid extraction container received in the container receiving portion.

The fourth support member and the mixing member are further disposed with respect each other such that when the fourth support member and the mixing member move horizontally relative to each other, at least two magnets in the mixing member have different heights relative to the bottom of the nucleic acid extraction container received in the container receiving portion.

As an implementation, the at least two magnets in the mixing member have different heights relative to the fourth support member; or
the at least two magnets in the mixing member are mounted at different heights on the magnet bracket, such that the at least two magnets in the mixing member have different heights relative to the fourth support member.

As an implementation, the second functional assembly further includes a third driving member, and the mixing member includes a movable magnet bracket and a plurality of magnets fixed to the magnet bracket, wherein the third driving member is configured to drive the magnet bracket to move so as to drive the plurality of magnets fixed to the magnet bracket to move relative to the nucleic acid extraction container on the fourth support member, such that the magnetic beads in the nucleic acid extraction container placed on the fourth support member perform a three-dimensional movement in the liquid in the nucleic acid extraction container under the effect of the moving magnets; or
the second functional assembly further includes a third driving member, the mixing member is configured to generate a magnetic field, and the third driving member is configured to drive relative movement between the fourth support member and the mixing member, such that the mixing member generates an alternately varying magnetic field relative to the nucleic acid extraction container on the fourth support member, enabling the magnetic beads in the nucleic acid extraction container to perform a three-dimensional movement in the liquid in the nucleic acid extraction container under the effect of the alternately varying magnetic field; or
the second functional assembly further includes a third driving member, the fourth support member includes at least a lysis zone, and the nucleic acid extraction container undergoes a lysis and capture stage in the lysis zone, where during the lysis and capture stage, cells in the sample in the nucleic acid extraction container are lysed to release nucleic acids, and the released nucleic acids are captured by the magnetic beads in the nucleic acid extraction container; the mixing member is configured to generate a magnetic field covering at least the lysis zone; and the third driving member is configured to drive relative movement between the fourth support member and the mixing member, such that the magnetic beads in the nucleic acid extraction container located in the lysis zone are moved so as to capture the released nucleic acids.

As an implementation, the sample analyzer further includes a frame assembly. The nucleic acid extraction device is mounted on the frame assembly.

The second functional assembly includes a fourth support member and a first driving member. The fourth support member is movably mounted on the frame assembly and configured to support the nucleic acid extraction container for performing an incubation operation, at least one first transfer station and a plurality of accommodating holes are formed on the fourth support member, with each accommodating hole being configured to accommodate one nucleic acid extraction container, and the first driving member is configured to drive the fourth support member to move relative to the frame assembly, such that the fourth support member drives each of the plurality of nucleic acid extraction containers to move to the first transfer station. The first support member is fixed to the frame assembly. The transfer assembly is configured to at least transfer the nucleic acid extraction container between the first transfer station and the first support member.

As an implementation, the sample analyzer further includes a frame assembly. The nucleic acid extraction device is mounted on the frame assembly.

The second functional assembly includes a fourth support member. The fourth support member is fixed to the frame assembly and configured to support the nucleic acid extraction container for performing an incubation operation, and at least one first transfer station and a plurality of accommodating holes are formed on the fourth support member, with each accommodating hole being configured to accommodate one nucleic acid extraction container. The first support member is fixed to the frame assembly. The transfer assembly is configured to at least transfer the nucleic acid extraction container between the first transfer station and the first support member, and is configured to be capable of performing at least a three-dimensional movement.

As an implementation, the sample analyzer further includes:
a container providing device configured to provide the nucleic acid extraction container and the amplification reaction container;
a reagent storage compartment configured to store a first-type reagent and a second-type reagent, the first-type reagent being configured to react with the sample to obtain a nucleic acid extract, and the second-type reagent being configured to react with the nucleic acid extract for amplification;
a reagent dispensing device configured to dispense the first-type reagent stored in the reagent storage compartment into the nucleic acid extraction container, and to dispense the second-type reagent stored in the reagent storage compartment into the amplification reaction container; and
a conveying device configured to at least convey the nucleic acid extraction container provided by the container providing device to the nucleic acid extraction device, and to convey the amplification reaction container provided by the container providing device to the amplification device.

A second object of the disclosure is to provide a sample analyzer, including:
a sample distribution device configured to aspirate a sample from a sample container and dispense at least part of the aspirated sample into a nucleic acid extraction container;
a nucleic acid extraction device configured to extract nucleic acids from a liquid containing at least the sample and a reagent in the nucleic acid extraction container to obtain a nucleic acid extract;
a pipetting device configured to transfer the nucleic acid extract from the nucleic acid extraction container to an amplification reaction container;
an amplification device configured to amplify the nucleic acid extract in the amplification reaction container to obtain a test solution; and
a test device configured to test the test solution,
wherein the nucleic acid extraction device includes a first functional assembly, a second functional assembly, and a transfer assembly, the first functional assembly being disposed above or below the second functional assembly, the transfer assembly being configured to transfer the nucleic acid extraction container between the first functional assembly and the second functional assembly, the first functional assembly being configured to at least support the nucleic acid extraction container for performing a first functional operation, and the second functional assembly being configured to at least support the nucleic acid extraction container for performing a second functional operation, so as to perform nucleic acid extraction.

The first functional operation and the second functional operation are either different functional operations or the same functional operation performed in sequence.

As an implementation, the first functional operation includes at least one of a liquid aspiration operation, a liquid dispensing operation, and a magnetic attraction operation; and/or
the second functional operation includes at least one of an incubation operation and a mixing operation.

As an implementation, the second functional assembly includes a fourth support member and a mixing member, the fourth support member being configured to support the nucleic acid extraction container for performing an incubation operation, and the mixing member being configured to mix the liquid containing at least the sample and the reagent in the nucleic acid extraction container located on the fourth support member,
wherein the mixing member includes at least a first magnet array and a second magnet array extending horizontally, with each magnet array including a plurality of magnets spaced apart and mounted on a magnet bracket.

The fourth support member includes a container receiving portion for placement of the nucleic acid extraction container.

The fourth support member and the mixing member are disposed with respect to each other such that when the nucleic acid extraction container is received in the container receiving portion, at least a bottom of the nucleic acid extraction container is located between the first magnet array and the second magnet array.

The fourth support member and the mixing member are capable of moving horizontally relative to each other such that the mixing member is capable of mixing the liquid containing at least the sample and magnetic beads in the nucleic acid extraction container received in the container receiving portion.

The fourth support member and the mixing member are further disposed with respect to each other such that when the fourth support member and the mixing member move horizontally relative to each other, at least two magnets in the mixing member have different heights relative to the bottom of the nucleic acid extraction container received in the container receiving portion.

As an implementation, the second functional assembly includes a fourth support member, a mixing member, and a third driving member, where the fourth support member is configured to support the nucleic acid extraction container for performing an incubation operation, the mixing member is configured to mix the liquid containing at least the sample and the reagent in the nucleic acid extraction container located on the fourth support member, the mixing member includes a movable magnet bracket and a plurality of magnets fixed to the magnet bracket, and the third driving member is configured to drive the magnet bracket to move so as to drive the plurality of magnets fixed to the magnet bracket to move relative to the nucleic acid extraction container on the fourth support member, such that magnetic beads in the nucleic acid extraction container placed on the fourth support member perform a three-dimensional movement in the liquid in the nucleic acid extraction container under the effect of the moving magnets; or
the second functional assembly includes a fourth support member, a mixing member, and a third driving member, where the fourth support member is configured to support the nucleic acid extraction container for performing an incubation operation, the mixing member is configured to generate a magnetic field to mix the liquid containing at least the sample and the reagent in the nucleic acid extraction container located on the fourth support member, and the third driving member is configured to drive relative movement between the fourth support member and the mixing member, such that the mixing member generates an alternately varying magnetic field relative to the nucleic acid extraction container on the fourth support member, enabling magnetic beads in the nucleic acid extraction container to perform a three-dimensional movement in the liquid in the nucleic acid extraction container under the effect of the alternately varying magnetic field; or
the second functional assembly includes a fourth support member, a mixing member, and a third driving member, where the fourth support member is configured to support the nucleic acid extraction container for performing an incubation operation, the fourth support member includes at least a lysis zone, and the nucleic acid extraction container undergoes a lysis and capture stage in the lysis zone, where during the lysis and capture stage, cells in the sample in the nucleic acid extraction container are lysed to release nucleic acids, and the released nucleic acids are captured by magnetic beads in the nucleic acid extraction container; the mixing member is configured to generate a magnetic field covering at least the lysis zone to mix at least the liquid containing at least the sample and the reagent in the nucleic acid extraction container located in the lysis zone; and the third driving member is configured to drive relative movement between the fourth support member and the mixing member, such that the magnetic beads in the nucleic acid extraction container located in the lysis zone are moved so as to capture the released nucleic acids.

A third object of the disclosure is to provide a sample analyzer, including:
a sample distribution device configured to aspirate a sample from a sample container and dispense at least part of the aspirated sample into a nucleic acid extraction container;
a nucleic acid extraction device configured to extract nucleic acids from a liquid containing at least the sample and a reagent in the nucleic acid extraction container to obtain a nucleic acid extract;
a pipetting device configured to transfer the nucleic acid extract from the nucleic acid extraction container to an amplification reaction container;
an amplification device configured to amplify the nucleic acid extract in the amplification reaction container to obtain a test solution; and
a test device configured to test the test solution,
wherein the nucleic acid extraction device has at least two stations, with each station being configured to support the nucleic acid extraction container for performing a processing operation for nucleic acid extraction, at least two of the stations being vertically arranged one above the other, and the processing operation including at least one of an incubation operation, a mixing operation, a magnetic attraction operation, a liquid aspiration operation, and a liquid dispensing operation.

As an implementation, performing nucleic acid extraction on a liquid containing at least the sample and a reagent by the nucleic acid extraction device includes: a process of performing lysis on the liquid containing at least the sample and the reagent, and performing post-lysis processing.

As an implementation, the nucleic acid extraction device includes a first-layer space and a second-layer space. The first-layer space is disposed above or below the second-layer space.

A liquid aspiration-dispensing station, a dedicated liquid aspiration station, and a dedicated liquid dispensing station are disposed in the first-layer space; and
a lysis incubation station, a cleaning incubation station, a drying incubation station, and an elution incubation station are disposed in the second-layer space,
wherein the lysis incubation station is configured to support the nucleic acid extraction container for performing a lysis incubation operation;
the cleaning incubation station is configured to support the nucleic acid extraction container for performing a cleaning incubation operation;
the drying incubation station is configured to support the nucleic acid extraction container for performing a drying incubation operation;
the elution incubation station is configured to support the nucleic acid extraction container for performing an elution incubation operation;
the liquid aspiration-dispensing station is configured to support the nucleic acid extraction container for performing a first liquid aspiration operation, and a first liquid dispensing operation for dispensing a cleaning liquid;
the dedicated liquid aspiration station is configured to support the nucleic acid extraction container for performing a second liquid aspiration operation; and
the dedicated liquid dispensing station is configured to support the nucleic acid extraction container for performing a second liquid dispensing operation for dispensing an elution reagent.

A fourth object of the disclosure is to provide a sample analyzer, including:
a sample distribution device configured to aspirate a sample from a sample container and dispense at least part of the aspirated sample into a nucleic acid extraction container;
a nucleic acid extraction device configured to perform a nucleic acid extraction operation on a liquid containing at least the sample and a reagent in the nucleic acid extraction container to obtain a nucleic acid extract;
a pipetting device configured to transfer the nucleic acid extract from the nucleic acid extraction container to an amplification reaction container;
an amplification device configured to amplify the nucleic acid extract in the amplification reaction container to obtain a test solution; and
a test device configured to test the test solution,
wherein the nucleic acid extraction device includes a lysis module, a cleaning module, an elution module, and a transfer assembly, at least two of the lysis module, the cleaning module and the elution module being arranged in a vertical direction, the lysis module being configured to support the nucleic acid extraction container for performing a lysis operation, the cleaning module being configured to support the nucleic acid extraction container for performing a cleaning operation, and the elution module being configured to support the nucleic acid extraction container for performing an elution operation; and the transfer assembly is configured to sequentially transfer the nucleic acid extraction container to the lysis module, the cleaning module, and the elution module.

As an implementation, performing nucleic acid extraction on a liquid containing at least the sample and a reagent by the nucleic acid extraction device includes: a process of performing lysis on the liquid containing at least the sample and the reagent, and performing post-lysis processing.

As an implementation, the nucleic acid extraction device further includes a drying module disposed below or above at least one of the lysis module, the cleaning module, and the elution module, the drying module being configured to support the nucleic acid extraction container for performing a drying operation, and the transfer assembly is configured to sequentially transfer the nucleic acid extraction container to the lysis module, the cleaning module, the drying module, and the elution module.

In the sample analyzer according to the first object of the disclosure, the first functional assembly of the nucleic acid extraction device that is configured to at least perform magnetic attraction and a liquid aspiration operation on the liquid in the nucleic acid extraction container is disposed above or below the second functional assembly configured to at least incubate the liquid in the nucleic acid extraction container, and the nucleic acid extraction container is transferred between the first functional assembly and the second functional assembly via the transfer assembly. That is, the first functional assembly and the second functional assembly, which are configured to perform different functional operations on the liquid in the nucleic acid extraction container, are vertically disposed one above the other. When the liquid in the nucleic acid extraction container needs to undergo an incubation operation, the transfer assembly transfers the nucleic acid extraction container to the second functional assembly. When the liquid in the nucleic acid extraction container needs to undergo magnetic attraction and a liquid aspiration operation, the transfer assembly transfers the nucleic acid extraction container to the first functional assembly. By adopting such a configuration, different functional assemblies of the nucleic acid extraction device may be arranged with full utilization of vertical space, thereby helping reduce the horizontal space occupied by the nucleic acid extraction device, facilitating a miniaturization design of the sample analyzer, and improving space utilization in a laboratory where the sample analyzer is located.

In the sample analyzer according to the second object of the disclosure, one of the first functional assembly and the second functional assembly of a nucleic acid extraction device that are configured to perform different functional operations or to perform the same functional operation in sequence is disposed above or below the other, and the nucleic acid extraction container is transferred between the first functional assembly and the second functional assembly via the transfer assembly. That is, the first functional assembly and the second functional assembly, which are configured to perform different functional operations or to perform the same functional operation in sequence on a liquid in the nucleic acid extraction container, are vertically disposed one above the other, and the transfer assembly transfers the nucleic acid extraction container to the first functional assembly and the second functional assembly, respectively. By adopting such a configuration, different functional assemblies of the nucleic acid extraction device may be arranged with full utilization of vertical space, thereby helping reduce the horizontal space occupied by the nucleic acid extraction device, facilitating a miniaturization design of the sample analyzer, and improving space utilization in a laboratory where the sample analyzer is located.

In the sample analyzer according to the third object of the disclosure, at least two stations in the nucleic acid extraction device are vertically arranged one above the other, with each station being configured to support the nucleic acid extraction container for performing at least one processing operation selected from an incubation operation, a mixing operation, a magnetic attraction operation, a liquid aspiration operation, and a liquid dispensing operation. That is, the at least two stations configured to support the nucleic acid extraction container for performing processing operations are vertically arranged one above the other. By adopting such a configuration, a plurality of stations in the nucleic acid extraction device may be arranged with full utilization of vertical space, thereby helping reduce the horizontal space occupied by the nucleic acid extraction device, facilitating a miniaturization design of the sample analyzer, and improving space utilization in a laboratory where the sample analyzer is located.

In the sample analyzer according to the fourth object of the disclosure, at least two of the lysis module, the cleaning module and the elution module of the nucleic acid extraction device are vertically arranged one above the other. That is, functional modules configured to support the nucleic acid extraction container for performing different functional operations in sequence are vertically arranged one above the other. By adopting such a configuration, a plurality of functional modules in the nucleic acid extraction device may be arranged with full utilization of vertical space, thereby helping reduce the horizontal space occupied by the nucleic acid extraction device, facilitating a miniaturization design of the sample analyzer, and improving space utilization in a laboratory where the sample analyzer is located.

### BRIEF DESCRIPTION OF THE DRAWINGS

To more clearly illustrate technical solutions in embodiments of the disclosure or in the prior art, the drawings required for describing the embodiments or the prior art are briefly described below. It is apparent that the drawings in the following description are merely some embodiments of the disclosure, and a person of ordinary skill in the art may still derive other drawings from the configurations shown in these drawings without involving creative effort.
FIG. 1 is a structural schematic diagram of a sample analyzer according to a first embodiment of the disclosure;
FIG. 2 is a structural schematic perspective view of a nucleic acid extraction device according to the first embodiment of the disclosure;
FIG. 3 is another structural schematic perspective view of the nucleic acid extraction device according to the first embodiment of the disclosure;
FIG. 4 is a schematic flowchart illustrating movement of a nucleic acid extraction container to different stations in a nucleic acid extraction device according to the first embodiment of the disclosure, with dashed arrows indicating a direction of travel of the nucleic acid extraction container between a plurality of stations;
FIG. 5 is a schematic distribution diagram of a first transfer station according to Embodiment I of the disclosure;
FIG. 6 is a schematic top view of a second functional assembly according to Embodiment I of the disclosure;
FIG. 7 is a structural schematic perspective view of a first functional assembly according to Embodiment I of the disclosure;
FIG. 8 is another structural schematic perspective view of the first functional assembly according to Embodiment I of the disclosure;
FIG. 9 is a structural schematic view of a first module according to Embodiment I of the disclosure;
FIG. 10 is a structural schematic view of a third module according to Embodiment I of the disclosure;
FIG. 11 is a schematic partial enlarged view of FIG. 10;
FIG. 12 is a structural schematic view of a fourth module according to Embodiment I of the disclosure;
FIG. 13 is a structural schematic view of a transfer assembly and the nucleic acid extraction container according to Embodiment I of the disclosure;
FIG. 14 is a structural schematic view of a first pipetting tip and a second pipetting tip according to Embodiment I of the disclosure;
FIG. 15 is a structural schematic perspective view of a mixing member according to Embodiment I of the disclosure;
FIG. 16 is a top schematic distribution diagram of a first magnet array and a second magnet array according to Embodiment I of the disclosure;
FIG. 17 is a developed schematic front view of the first magnet array and the second magnet array according to Embodiment I of the disclosure;
FIG. 18 is a developed schematic top view of the first magnet array and the second magnet array according to Embodiment I of the disclosure; and
FIG. 19 is a schematic distribution diagram of a first transfer station according to a second embodiment of the disclosure.

### DESCRIPTION OF THE PREFERRED EMBODIMENTS

The technical solutions in the embodiments of the disclosure will be described below clearly and completely in conjunction with the accompanying drawings in the embodiments of the disclosure. Obviously, the embodiments described are merely some rather than all of the embodiments of the disclosure. Based on the embodiments in the disclosure, all other embodiments obtained by a person of ordinary skill in the art without involving creative effort shall fall within the scope of protection of the disclosure.

In addition, the technical solutions of the various embodiments may be combined, provided that such combinations are achievable by a person of ordinary skill in the art. When a combination of technical solutions is contradictory or is not feasible, such a combination of technical solutions shall be considered non-existent and shall not fall within the scope of protection of the disclosure.

This embodiment mainly optimizes the layout of a nucleic acid extraction device in a sample analyzer, making full use of vertical space for a vertical layout, thereby solving the problem of the nucleic acid extraction device occupying excessive planar area in the sample analyzer, and further solving the problem of a large overall footprint of the sample analyzer.

### First embodiment:

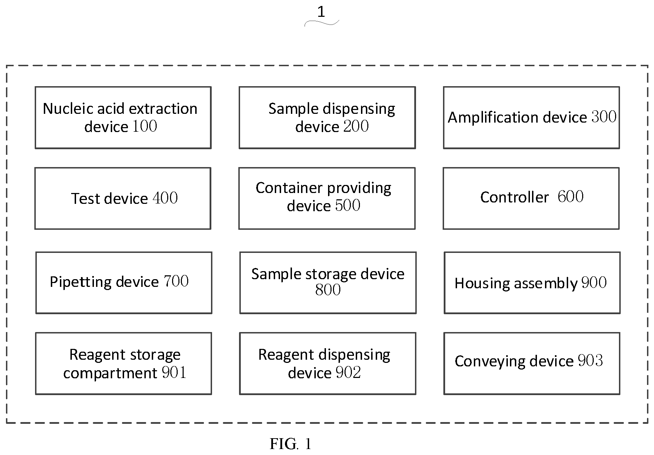

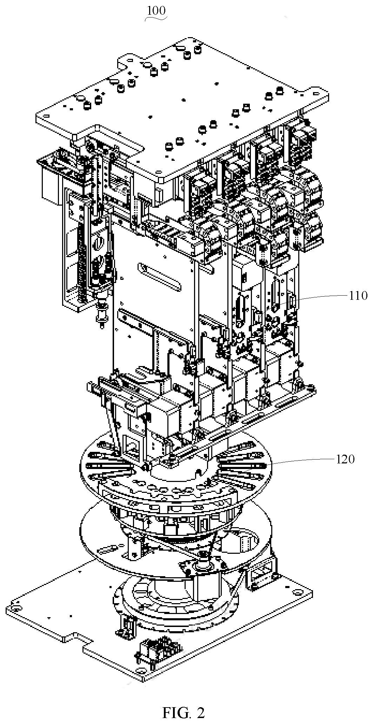

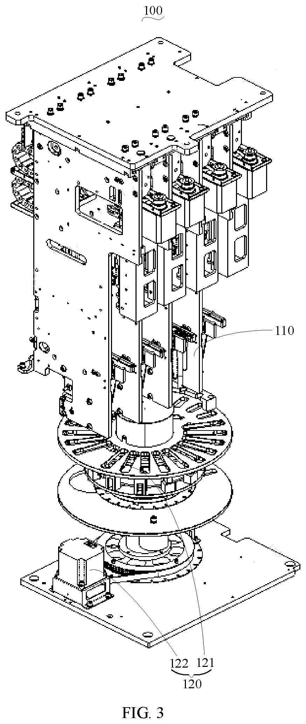

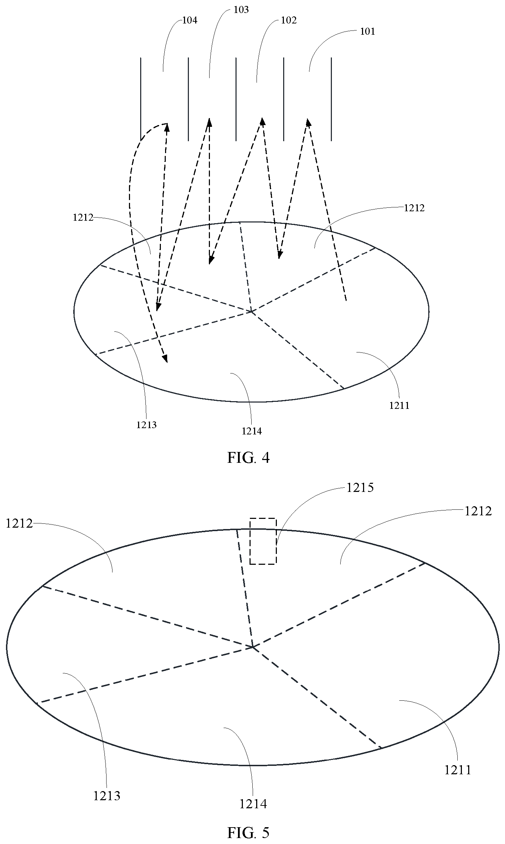

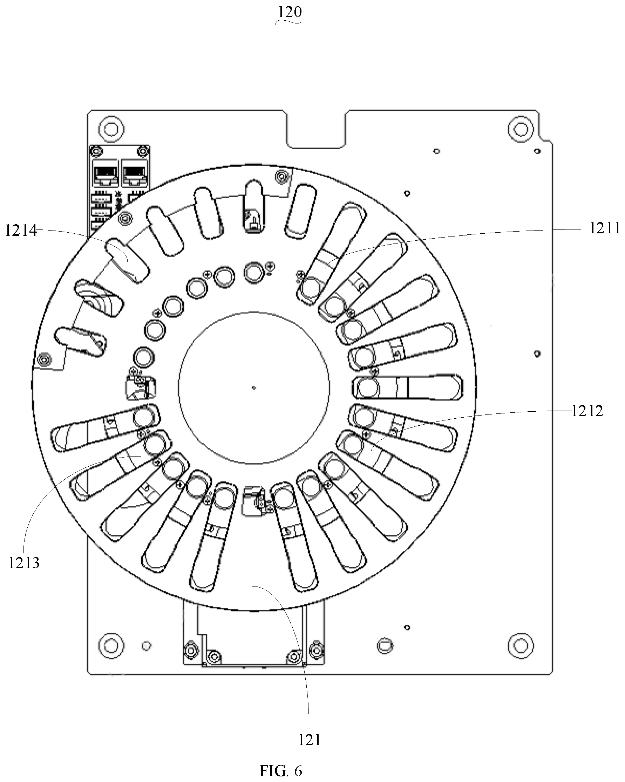

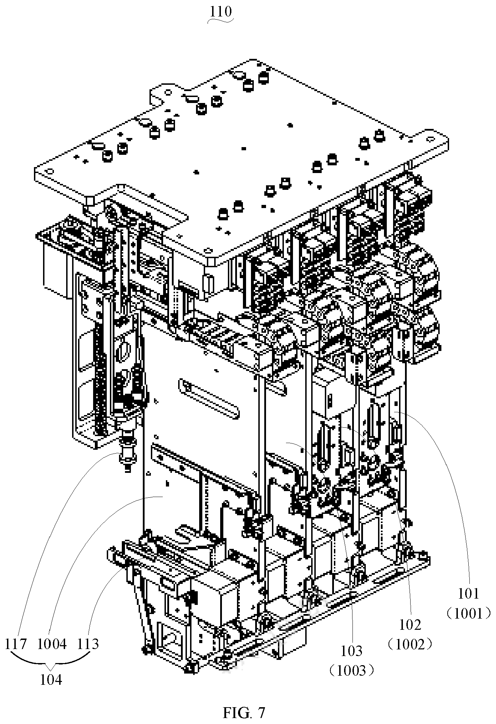

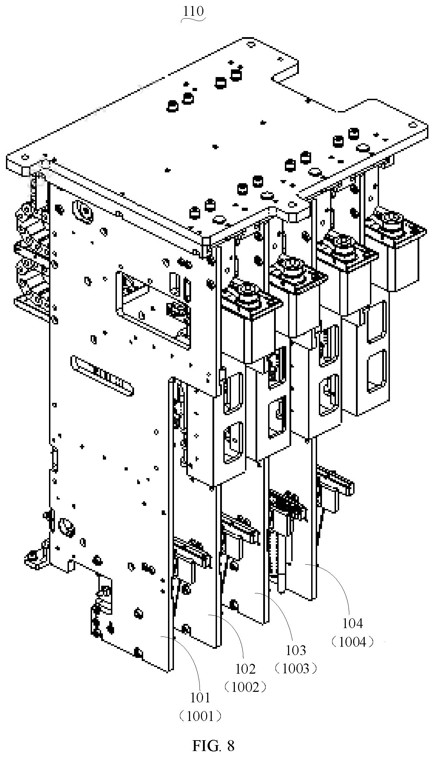

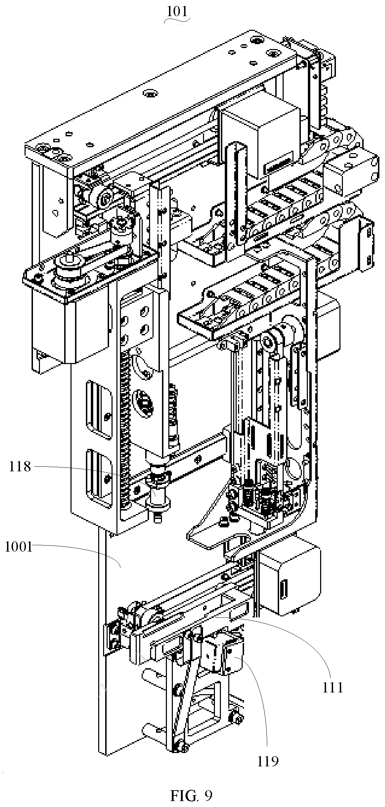

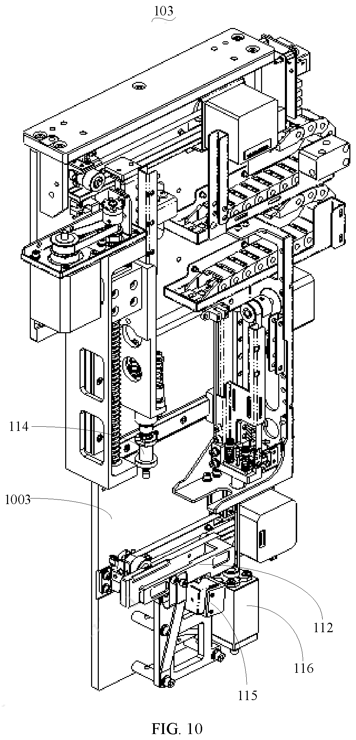

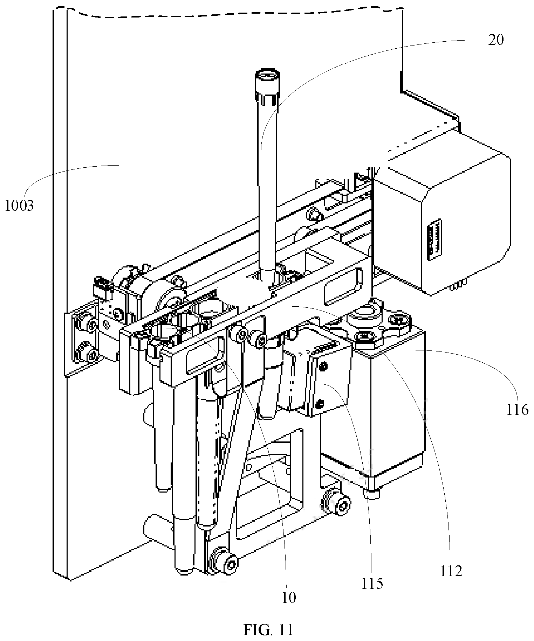

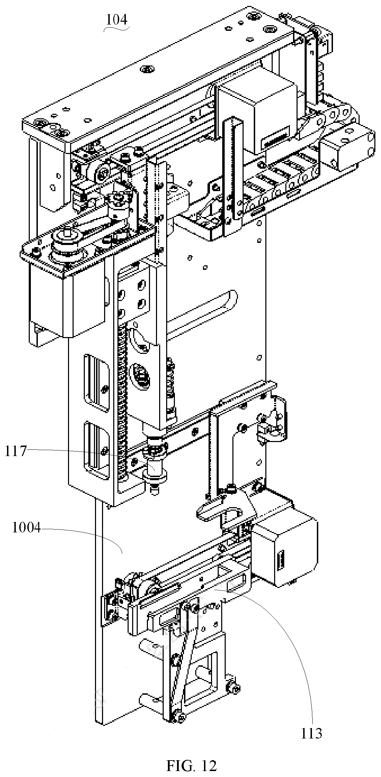

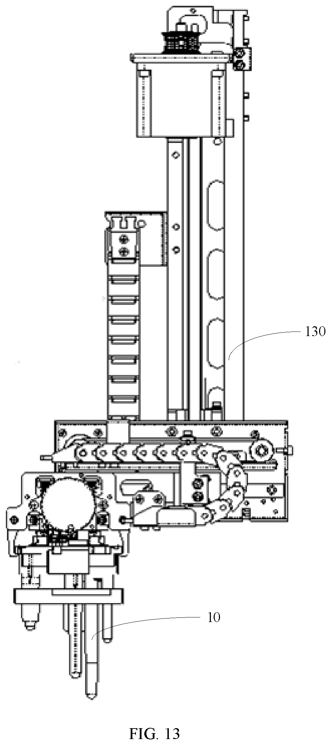

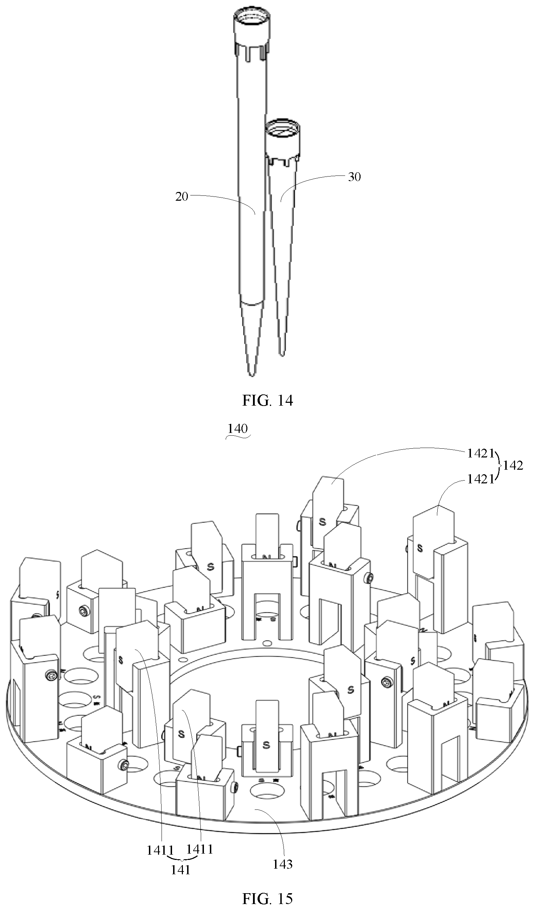

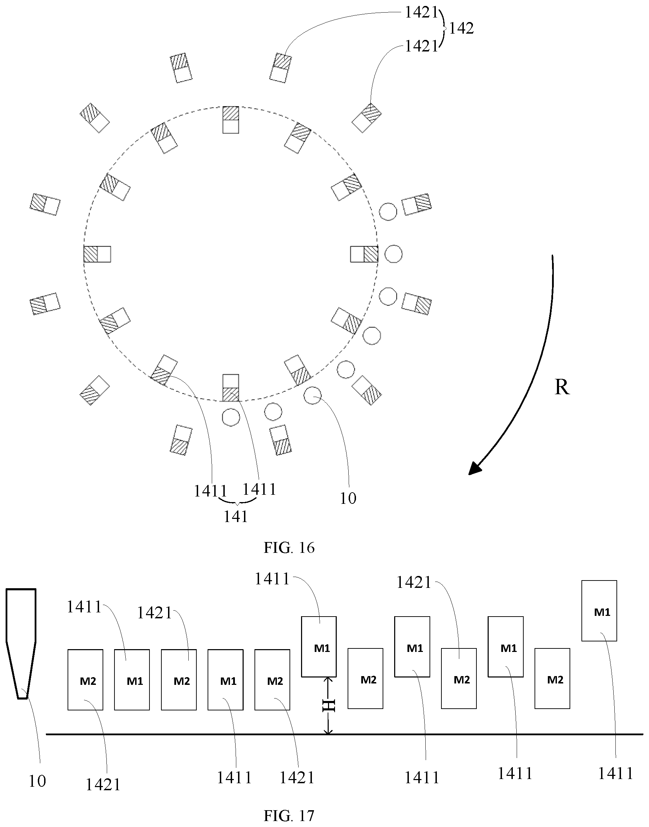

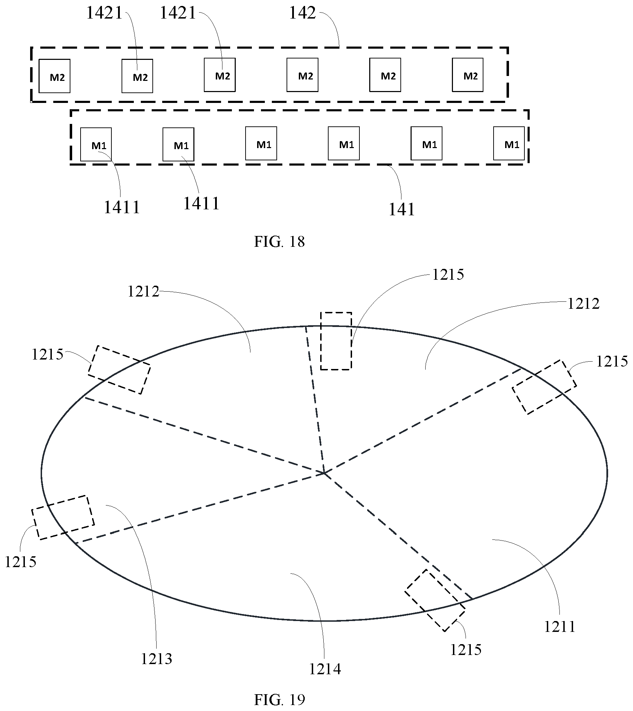

As shown in FIGS. 1 to 18, a sample analyzer 1 according to the first embodiment of the disclosure includes a sample distribution device 200, a nucleic acid extraction device 100, an amplification device 300, and a test device 400. The sample distribution device 200 is configured to dispense a sample. The nucleic acid extraction device 100 is configured to extract nucleic acids from a liquid containing at least the sample to obtain a nucleic acid extract. The amplification device 300 is configured to amplify a liquid containing at least the nucleic acid extract to obtain a test solution. The test device 400 is configured to test the test solution. The nucleic acid extraction device 100 is primarily configured to extract nucleic acids from the sample to obtain purified nucleic acids. The amplification device 300 is configured to amplify the nucleic acids extracted by the nucleic acid extraction device, so as to substantially increase the amount of nucleic acids within a relatively short period of time. The sample analyzer 1 according to this embodiment is primarily configured to obtain a test solution by performing nucleic acid extraction and amplification on the sample, and then to test the test solution to obtain a test result of the sample.

As an implementation, the nucleic acid extraction device 100 extracts nucleic acids from a biological sample stored in a nucleic acid extraction container 10 by a magnetic bead method. The amplification device 300 is configured to amplify the nucleic acids extracted by the nucleic acid extraction device 100, so as to substantially increase the amount of nucleic acids within a relatively short period of time. The test device 400 is configured to test the amplified nucleic acids.

As an implementation, the nucleic acid extraction device 100 includes a first functional assembly 110, a second functional assembly 120, and a transfer assembly 130. The first functional assembly 110 is disposed above or below the second functional assembly 120, and the transfer assembly 130 is configured to transfer the nucleic acid extraction container 10 between the first functional assembly 110 and the second functional assembly 120, to perform nucleic acid extraction to obtain a nucleic acid extract. The first functional assembly 110 is configured to at least perform magnetic attraction and a liquid aspiration operation on the liquid in the nucleic acid extraction container 10, and the second functional assembly 120 is configured to at least incubate the liquid in the nucleic acid extraction container 10. The first functional assembly 110 and the second functional assembly 120 are two different functional assemblies of the nucleic acid extraction device 100. The first functional assembly 110 is primarily configured to perform magnetic attraction and a liquid aspiration operation on the liquid in the nucleic acid extraction container 10, and the second functional assembly 120 is primarily configured to incubate the liquid in the nucleic acid extraction container 10. In this embodiment, the first functional assembly 110 and the second functional assembly 120, which are configured to perform different functional operations on the liquid in the nucleic acid extraction container 10, are vertically disposed one above the other. The transfer assembly 130 transfers the nucleic acid extraction container 10 vertically between the first functional assembly 110 and the second functional assembly 120. When the liquid in the nucleic acid extraction container 10 needs to undergo an incubation operation, the transfer assembly 130 transfers the nucleic acid extraction container 10 to the second functional assembly 120. When the liquid in the nucleic acid extraction container 10 needs to undergo magnetic attraction and a liquid aspiration operation, the transfer assembly 130 transfers the nucleic acid extraction container 10 to the first functional assembly 110. By adopting such a configuration, different functional assemblies of the nucleic acid extraction device 100 may be arranged with full utilization of vertical space, thereby helping reduce the horizontal space occupied by the nucleic acid extraction device 100, facilitating a miniaturization design of the sample analyzer 1, and improving space utilization in a laboratory where the sample analyzer 1 is located.

As an implementation, the sample analyzer 1 further includes a pipetting device 700. The sample distribution device 200 is configured to aspirate at least part of a sample from a sample container and dispense same into the nucleic acid extraction container 10 provided by a second consumable supply device. The nucleic acid extraction device 100 is configured to extract nucleic acids from a liquid containing at least the sample and a reagent in the nucleic acid extraction container 10 to obtain a nucleic acid extract. The reagent contains magnetic beads. The pipetting device 700 is configured to transfer the nucleic acid extract from the nucleic acid extraction container 10 to an amplification reaction container. The amplification device 300 is configured to amplify a liquid containing at least the nucleic acid extract in the amplification reaction container to obtain a test solution. The sample container, the nucleic acid extraction container 10, and the amplification reaction container are three different types of containers. The pipetting device 700 is primarily configured to transfer the nucleic acid extract between the nucleic acid extraction container 10 and the amplification reaction container. The sample container is primarily configured to accommodate a sample collected from a patient, that is, the sample container is primarily configured to provide an accommodating site for the sample. The nucleic acid extraction container 10 is primarily configured to receive the sample for nucleic acid extraction, that is, the nucleic acid extraction container 10 is primarily configured to provide a nucleic acid extraction site for the sample. The amplification reaction container is primarily configured to receive the nucleic acid extract for an amplification reaction, that is, the amplification reaction container is primarily configured to provide an amplification reaction site for the nucleic acid extract. The nucleic acid extraction container 10 and the amplification reaction container are two different consumables of the sample analyzer 1, such that the nucleic acid extraction container 10 may be designed into a shape that facilitates nucleic acid extraction according to requirements, and the amplification reaction container may be designed into a shape that facilitates amplification reaction according to requirements.

As an implementation, the first functional assembly 110 includes a first support member 111, a first pipetting member 118, and a first magnetic attraction member 119. The first support member 111 is configured to support the nucleic acid extraction container 10. The first magnetic attraction member 119 is configured to perform a first magnetic attraction on the liquid containing at least the sample and the reagent in the nucleic acid extraction container 10 located on the first support member 111 so as to attract the magnetic beads in the nucleic acid extraction container 10. The first pipetting member 118 is configured to perform a first liquid aspiration operation on the liquid in the nucleic acid extraction container 10 located on the first support member 111 and subjected to the first magnetic attraction by the first magnetic attraction member 119. The transfer assembly 130 is configured to transfer the nucleic acid extraction container 10 between the first support member 111 and the second functional assembly 120. The magnetic attraction includes the first magnetic attraction, and the liquid aspiration operation includes the first liquid aspiration operation. The first magnetic attraction member 119 includes at least one magnet. During incubation, nucleic acids and magnetic beads in the biological sample bind together. The first magnetic attraction member 119 is primarily configured to perform a first magnetic attraction on the liquid in the nucleic acid extraction container 10 placed on the first support member 111, where the first magnetic attraction enables attraction of complexes of magnetic beads and nucleic acids. The first pipetting member 118 is primarily configured to aspirate waste liquid from the nucleic acid extraction container 10 and discharge the waste liquid. That is, the first pipetting member 118 is primarily configured to aspirate the liquid in the nucleic acid extraction container 10 other than the magnetic beads and the complexes of magnetic beads and nucleic acids, and discharge same as waste liquid, thereby facilitating nucleic acid purification.

As an implementation, the first functional assembly 110 further includes a liquid dispensing member configured to perform a first liquid dispensing operation for dispensing a cleaning liquid into the nucleic acid extraction container 10 located on the first support member 111. The liquid dispensing member is primarily configured to add the cleaning liquid into the nucleic acid extraction container 10 to facilitate a subsequent cleaning function.

In particular, the liquid dispensing member is primarily configured to perform a first liquid dispensing operation to add the cleaning liquid into the nucleic acid extraction container 10 that has been subjected to the first liquid aspiration operation, and the added cleaning liquid is used for cleaning the liquid in the nucleic acid extraction container 10. The second functional assembly 120 is configured to support the nucleic acid extraction container 10 for performing an incubation operation. Specifically, the second functional assembly 120 is configured to support the nucleic acid extraction container 10 for performing a lysis incubation operation and a washing incubation operation. The transfer assembly 130 is configured to transfer the nucleic acid extraction container 10 that has been subjected to the lysis incubation operation from the second functional assembly 120 to the first support member 111, and to transfer the nucleic acid extraction container 10 that has been sequentially subjected to the first liquid aspiration operation and the first liquid dispensing operation from the first support member 111 to the second functional assembly 120 to perform the washing incubation operation.

As an implementation, at least two first support members 111 are provided. Each first support member 111 is correspondingly provided with at least one liquid dispensing member and at least one first pipetting member 118. The at least two first support members 111 form at least two stations disposed in sequence. After one of the first support members 111 completes a first magnetic attraction, a first liquid aspiration operation, and a first liquid dispensing operation, the nucleic acid extraction container 10 is first conveyed to the second functional assembly 120 for cleaning incubation, and then conveyed to the next first support member 111 to undergo a next first magnetic attraction, a next first liquid aspiration operation, and a next first liquid dispensing operation. In this embodiment, the nucleic acid extraction device 100 performs at least two rounds of magnetic attraction, waste liquid aspiration, and cleaning liquid dispensing on the liquid in the nucleic acid extraction container 10. Accordingly, the liquid in the nucleic acid extraction container 10 undergoes at least two rounds of cleaning and magnetic separation, thereby facilitating the removal and discharge of non-nucleic acid components from the sample and ensuring the purification degree of the nucleic acids. Certainly, in practical applications, as an alternative embodiment, it is possible to provide only one first support member 111.

As an implementation, the number of liquid dispensing members and the number of first pipetting members 118 are the same as the number of first support members 111, that is, each first support member 111 is correspondingly provided with one liquid dispensing member and one first pipetting member 118. In this way, a plurality of nucleic acid extraction containers 10 may operate in parallel on the first functional assembly 110, and the movement spans of the liquid dispensing members and the first pipetting members 118 may be reduced. Certainly, in practical applications, as an alternative embodiment, two first support members 111 may share the same liquid dispensing member and/or the same first pipetting member 118.

As an implementation, the number of first support members 111, the number of liquid dispensing members, and the number of first pipetting members 118 are all two. Certainly, in practical applications, the number of first support members 111, the number of liquid dispensing members, and the number of first pipetting members 118 may be three or more.

As an implementation, the first functional assembly 110 further includes a second support member 112, a second pipetting member 114, and a second magnetic attraction member 115. The second support member 112 is configured to support the nucleic acid extraction container 10. The second magnetic attraction member 115 is configured to perform a second magnetic attraction on the liquid containing at least the cleaning liquid in the nucleic acid extraction container 10 located on the second support member 112. The second pipetting member 114 is configured to perform a second liquid aspiration operation on the liquid in the nucleic acid extraction container 10 located on the second support member 112 and subjected to the second magnetic attraction by the second magnetic attraction member 115. The transfer assembly 130 is further configured to transfer the nucleic acid extraction container 10 between the second support member 112 and the second functional assembly 120. The magnetic attraction further includes the second magnetic attraction, and the liquid aspiration operation further includes the second liquid aspiration operation. The first support member 111 and the second support member 112 form two stations disposed in sequence. The nucleic acid extraction container 10 is conveyed to the second support member 112 to perform the second magnetic attraction and the second liquid aspiration operation only after all the first support members 111 have sequentially completed the first magnetic attraction, the first liquid aspiration operation, and the first liquid dispensing operation. The second pipetting member 114 is configured to perform a second liquid aspiration operation on the nucleic acid extraction container 10 located on the second support member 112 and having been sequentially subjected to all the first liquid aspiration operations and the first liquid dispensing operations.

Unlike the station formed by the first support member 111, the station formed by the second support member 112 is configured to perform only magnetic attraction and waste liquid aspiration and discharge, without the need to perform liquid dispensing. In contrast, the station formed by the first support member 111, after completing the magnetic attraction and waste liquid aspiration and discharge, is further required to perform an operation of dispensing the cleaning liquid.

As an implementation, the sample analyzer 1 further includes a first lifting member 116. The first lifting member 116 is connected to the second magnetic attraction member 115, and is configured to drive the second magnetic attraction member 115 to move up and down relative to the nucleic acid extraction container 10. In this embodiment, the second magnetic attraction member 115 moves downward to draw the magnetic complexes (the complexes of magnetic beads and nucleic acids) in the nucleic acid extraction container 10 downward, thereby enabling the magnetic complexes to aggregate at the bottom of the nucleic acid extraction container 10. This is conducive to avoiding an adverse condition in which the magnetic complexes are excessively dispersed such that an elution reagent fails to cover the magnetic complexes, thereby ensuring the reliability of a subsequent elution operation, and preventing the second pipetting member 114 from contacting or carrying away the magnetic complexes during liquid aspiration.

In particular, the first lifting member 116 is configured to drive the second magnetic attraction member 115 to move downward after the transfer assembly 130 places the nucleic acid extraction container 10 on the second support member 112 and before the second pipetting member 114 performs the second liquid aspiration operation. The second functional assembly 120 is further configured to support the nucleic acid extraction container 10 for performing a drying incubation operation. The transfer assembly 130 is further configured to transfer the nucleic acid extraction container 10 that has been subjected to the washing incubation operation from the second functional assembly 120 to the second support member 112, and to transfer the nucleic acid extraction container 10 that has been subjected to the second liquid aspiration operation from the second support member 112 to the second functional assembly 120 to perform the drying incubation operation.

As an implementation, the first functional assembly 110 further includes a third support member 113 and a third pipetting member 117. The third support member 113 is configured to support the nucleic acid extraction container 10, and the third pipetting member 117 is configured to perform a second liquid dispensing operation for dispensing an elution reagent into the nucleic acid extraction container 10 located on the third support member 113. The transfer assembly 130 is further configured to transfer the nucleic acid extraction container 10 between the third support member 113 and the second functional assembly 120. The first support member 111, the second support member 112, and the third support member 113 form three stations disposed in sequence. The nucleic acid extraction container 10 is conveyed to the third support member 113 to perform the second liquid dispensing operation only after all the first support members 111 have sequentially completed the first magnetic attraction, the first liquid aspiration operation, and the first liquid dispensing operation, and after the second support member 112 has completed the second magnetic attraction and the second liquid aspiration operation. The station formed by the third support member 113 is configured to perform only a liquid dispensing operation for dispensing the elution reagent, without the need to perform magnetic attraction and waste liquid aspiration and discharge actions.

In particular, the third pipetting member 117 is configured to perform the second liquid dispensing operation for dispensing the elution reagent into the nucleic acid extraction container 10 located on the third support member 113 and having been subjected to the second liquid aspiration operation. The second functional assembly 120 is further configured to support the nucleic acid extraction container 10 for performing an elution incubation operation. The transfer assembly 130 is further configured to transfer the nucleic acid extraction container 10 that has been subjected to the drying incubation operation from the second functional assembly 120 to the third support member 113, and to transfer the nucleic acid extraction container 10 that has been subjected to the second liquid dispensing operation from the third support member 113 to the second functional assembly 120 to perform the elution incubation operation.

As an implementation, the first support member 111, the second support member 112, and the third support member 113 are disposed side-by-side in a horizontal direction. That is, the first support member 111, the second support member 112, and the third support member 113 are disposed in a row in the horizontal direction, thereby facilitating simplification of the movement trajectory of the transfer assembly 130.

As an implementation, the second support member 112 is disposed between the first support member 111 and the third support member 113 in the horizontal direction.

As an implementation, two first support members 111, the second support member 112, and the third support member 113 are sequentially disposed side-by-side in the horizontal direction.

As an implementation, a reaction well is formed in the nucleic acid extraction container 10, and is configured to receive a sample and a reagent for nucleic acid extraction. The sample distribution device 200 is configured to aspirate a sample from a sample container and dispense at least part of the aspirated sample into the reaction well. The nucleic acid extraction device 100 is configured to extract nucleic acids from a liquid containing at least the sample and the reagent in the reaction well. The pipetting device 700 is configured to transfer a nucleic acid extract from the reaction well to the amplification reaction container.

As an implementation, at least one tip well is further formed in the nucleic acid extraction container 10. Each tip well is configured to accommodate one pipetting tip. The nucleic acid extraction device 100 is further configured to perform pipetting via at least one pipetting tip on the nucleic acid extraction container 10. Upon completion of a pipetting operation corresponding to a test item, a pipetting tip is released together with the nucleic acid extraction container 10 to a recovery station for recovery, thereby eliminating a cleaning stage and improving the testing efficiency. In addition, the pipetting tips are directly placed in the nucleic acid extraction container 10. This can eliminate the need to separately provide a storage compartment and a conveying mechanism for the pipetting tips in the sample analyzer 1. Moreover, this helps reduce the conveyance distance of the pipetting tips during pipetting using the pipetting tip, thereby improving the pipetting efficiency.

As an implementation, a first tip well is further formed in the nucleic acid extraction container 10, and is configured to accommodate a first pipetting tip 20. The first pipetting member 118 is configured to perform a first liquid aspiration operation on the liquid in the reaction well via the first pipetting tip 20. In this embodiment, the first pipetting member 118 aspirates waste liquid during the nucleic acid extraction process via a pipetting tip, thereby eliminating a needle-washing step of the first pipetting member 118, facilitating an improvement in the efficiency of nucleic acid extraction, and avoiding adverse cross-contamination caused by insufficient cleaning.

As an implementation, the first pipetting member 118 includes a first tip connecting component and a first aspiration-discharge drive component. The first tip connecting component is configured to be removably connected to the first pipetting tip 20 to perform liquid aspiration by the first pipetting tip 20 under power provided by the first aspiration-discharge drive component. The first tip connecting component is primarily configured to be in plug-in engagement with the first pipetting tip 20 to achieve gripping of the first pipetting tip 20. The first aspiration-discharge drive component is primarily configured to provide a driving force for liquid aspiration and discharge by the first pipetting tip 20.

As an implementation, the first pipetting member 118 further includes a first movement drive component configured to drive the first tip connecting component to move, such that the first tip connecting component is moved to different stations, such as a standby station, a tip gripping station, a liquid aspiration station, a liquid discharge station, and a tip release station.

As an implementation, the first movement drive component is configured to drive the first tip connecting component to move, such that the first tip connecting component is moved above the first tip well and is connected to the first pipetting tip 20, and the first tip connecting component drives the first pipetting tip 20 to move and insert into the reaction well. The first aspiration-discharge drive component is configured to provide a driving force for the first pipetting tip 20 to perform a liquid aspiration operation after the first pipetting tip 20 is inserted into the reaction well.

As an implementation, a second tip well is further formed in the nucleic acid extraction container 10 and is configured to accommodate a second pipetting tip 30. The third pipetting member 117 is configured to perform a second liquid dispensing operation on the reaction well via the second pipetting tip 30. In this embodiment, the third pipetting member 117 aspirates waste liquid during the nucleic acid extraction process via a pipetting tip, thereby eliminating a needle-washing step of the third pipetting member 117, and facilitating an improvement in the efficiency of nucleic acid extraction.

As an implementation, a capacity of the first pipetting tip 20 is greater than a capacity of the second pipetting tip 30. The first pipetting tip 20 is configured to aspirate and discharge the waste liquid, with a relatively large aspirated volume. The second pipetting tip 30 is configured to aspirate and discharge the elution reagent, with a relatively small aspirated volume. In this case, setting the capacity of the first liquid aspiration tip to be greater than the capacity of the second pipetting tip 30 enables the first liquid aspiration tip to aspirate the waste liquid from the reaction well in a single operation, thereby reducing the number of liquid aspiration and discharge operations of the first liquid aspiration tip and thus ensuring the efficiency of waste liquid aspiration and discharge, while also ensuring the accuracy of aspiration and dispensing of the elution reagent.

As an implementation, the third pipetting member 117 includes a second tip connecting component and a second aspiration-discharge drive component. The second tip connecting component is configured to be removably connected to the second pipetting tip 30 so as to perform liquid aspiration via the second pipetting tip 30 under power provided by the second aspiration-discharge drive component.

As an implementation, the third pipetting member 117 further includes a second movement drive component configured to drive the second tip connecting component to move, such that the second tip connecting component is moved to different stations, such as a standby station, a tip gripping station, a liquid aspiration station, a liquid discharge station, and a tip release station.

In particular, the second movement drive component is configured to drive the second tip connecting component to move, such that the second tip connecting component is moved above the second tip well and is connected to the second pipetting tip 30, the second tip connecting component drives the second pipetting tip 30 to move to a liquid aspiration station for the elution reagent, and the second tip connecting component drives the second pipetting tip 30 to move and insert into the reaction well. The second aspiration-discharge drive component is configured to provide a driving force for the second pipetting tip 30 to perform a liquid aspiration operation after the second pipetting tip 30 is moved to the liquid aspiration station for the elution reagent, and to provide a driving force for the second pipetting tip 30 to perform a second liquid dispensing operation after the second pipetting tip 30 is inserted into the reaction well.

As an implementation, a waste liquid well is further formed in the nucleic acid extraction container 10, and the first pipetting member 118 and the second pipetting member 114 are further configured to dispense liquid aspirated from the reaction well into the waste liquid well. In this embodiment, the waste liquid well is directly formed in the nucleic acid extraction container 10, thereby shortening the discharge path of the waste liquid during the nucleic acid extraction process.

As an implementation, the second pipetting member 114 includes a third tip connecting component, a third aspiration-discharge drive component, and a third movement drive component. The third tip connecting component is configured to connect to the first pipetting tip 20. The third movement drive component is configured to drive the third tip connecting component to move, such that the third tip connecting component is moved above the first tip well and is connected to the first pipetting tip 20, and the third tip connecting component drives the first pipetting tip 20 to move and insert into the reaction well. The third aspiration-discharge drive component is configured to provide a driving force for the first pipetting tip 20 to perform a liquid aspiration operation after the first pipetting tip 20 is inserted into the reaction well.

As an implementation, the liquid dispensing member includes a liquid dispensing needle, a liquid dispensing pipeline, and a fourth aspiration-discharge drive component. The liquid dispensing pipeline is configured to connect the liquid dispensing needle to a cleaning liquid container. The fourth aspiration-discharge drive component is configured to drive the liquid dispensing pipeline to aspirate a cleaning liquid from the cleaning liquid container, deliver the cleaning liquid to the liquid dispensing needle, and dispense the cleaning liquid through the liquid dispensing needle into the reaction well of the nucleic acid extraction container 10 located on the first support member 111.

As an implementation, the sample analyzer 1 further includes a frame assembly. The first functional assembly 110 includes a first connecting plate 1001, a second connecting plate 1002, a third connecting plate 1003, a fourth connecting plate 1004, two first support members 111, two first pipetting members 118, two liquid dispensing members, two first magnetic attraction members 119, one second support member 112, one second pipetting member 114, one second magnetic attraction member 115, one third support member 113, and one third pipetting member 117. The first connecting plate 1001 is connected to one of the first support members 111, one of the first pipetting members 118, one of the liquid dispensing members, and one of the first magnetic attraction members 119 to form a first module 101, and the first module 101 is connected to the frame assembly via the first connecting plate 1001. The second connecting plate 1002 is connected to the other first support member 111, the other first pipetting member 118, the other liquid dispensing member, and the other first magnetic attraction member 119 to form a second module 102, and the second module 102 is connected to the frame assembly via the second connecting plate 1002. The third connecting plate 1003 is connected to the second support member 112, the second pipetting member 114, and the second magnetic attraction member 115 to form a third module 103, and the third module 103 is connected to the frame assembly via the third connecting plate 1003. The fourth connecting plate 1004 is connected to the third support member 113 and the third pipetting member 117 to form a fourth module 104, and the fourth module 104 is connected to the frame assembly via the fourth connecting plate 1004.

As an implementation, the first module 101, the second module 102, the third module 103, and the fourth module 104 are sequentially disposed side-by-side in the horizontal direction.

As an implementation, the first module 101, the second module 102, the third module 103, and the fourth module 104 are sequentially disposed side-by-side along a linear trajectory.

As an implementation, the second functional assembly 120 includes a fourth support member 121 and a heating member. The fourth support member 121 is configured to support the nucleic acid extraction container 10 for performing an incubation operation. The heating member is configured to heat the liquid containing at least the sample and the reagent in the nucleic acid extraction container 10 located on the fourth support member 121. The heating member is primarily configured to provide an appropriate incubation ambient temperature for the liquid in the nucleic acid extraction container 10, thereby ensuring the incubation efficiency.

As an implementation, the fourth support member 121 includes a lysis zone 1211, a washing zone 1212, a drying zone 1213, and an elution zone 1214. An accommodating hole for placement of the nucleic acid extraction container 10 is formed in each of the lysis zone 1211, the washing zone 1212, the drying zone 1213, and the elution zone 1214. The transfer assembly 130 is configured to transfer the nucleic acid extraction container 10 between the lysis zone 1211, the washing zone 1212 and the first support member 111, between the washing zone 1212, the drying zone 1213 and the second support member 112, and between the drying zone 1213, the elution zone 1214 and the third support member 113. In particular, the transfer assembly 130 is configured to: transfer the nucleic acid extraction container 10 located in the lysis zone 1211 to the first support member 111, transfer the nucleic acid extraction container 10 located on the first support member 111 to the washing zone 1212, transfer the nucleic acid extraction container 10 located in the washing zone 1212 to the second support member 112, transfer the nucleic acid extraction container 10 located on the second support member 112 to the drying zone 1213, transfer the nucleic acid extraction container 10 located in the drying zone 1213 to the third support member 113, and transfer the nucleic acid extraction container 10 located on the third support member 113 to the elution zone 1214. In this embodiment, nucleic acid extraction performed by the nucleic acid extraction device 100 mainly includes lysis, washing, drying, and elution stages. The lysis stage is performed in the lysis zone 1211. In the lysis stage, cells in the sample are mainly lysed by a lysis reagent to release nucleic acids, and the released nucleic acids are attracted onto the magnetic beads in the nucleic acid extraction container 10. The washing stage is performed in the washing zone 1212. In the washing stage, a cleaning liquid (or referred to as a washing liquid) is mainly used to remove undesired components of the sample such as various impurities remaining on and between the magnetic beads, including proteins, lipids, and other substances. The drying stage is performed in the drying zone 1213. In the drying stage, the residual liquid after washing is dried mainly by heating. The elution stage is performed in the elution zone 1214. In the elution stage, the nucleic acids are mainly separated from the magnetic beads by the elution reagent, thereby obtaining purified nucleic acids. Certainly, in practical applications, the nucleic acid extraction step is not limited thereto. For example, as an alternative embodiment, the drying zone 1213 and the drying stage may be omitted.

As an implementation, two washing zones 1212 are formed on the fourth support member 121, namely, a first washing zone 1212 and a second washing zone 1212. The first functional assembly 110 includes two first support members 111. The transfer assembly 130 transferring the nucleic acid extraction container 10 located in the lysis zone 1211 to the first support member 111, transferring the nucleic acid extraction container 10 located on the first support member 111 to the washing zone 1212, and transferring the nucleic acid extraction container 10 located in the washing zone 1212 to the second support member 112 includes: transferring the nucleic acid extraction container 10 that has been subjected to lysis incubation from the lysis zone 1211 to one of the first support members 111; transferring the nucleic acid extraction container 10 that has been subjected to a first liquid aspiration operation and a first liquid dispensing operation from the first support member 111 to the first washing zone 1212; transferring the nucleic acid extraction container 10 that has been subjected to cleaning incubation from the first washing zone 1212 to the other first support member 111; transferring the nucleic acid extraction container 10 that has been subjected to a first liquid aspiration operation and a first liquid dispensing operation from the other first support member 111 to the second washing zone 1212; and transferring the nucleic acid extraction container 10 that has been subjected to cleaning incubation from the second washing zone 1212 to the second support member 112. In this embodiment, two washing zones 1212 are provided, thereby ensuring the cleanliness of the cleaning process and thus ensuring the purification degree of the nucleic acids. Certainly, in practical applications, the number of washing zones 1212 is not limited thereto, and may, for example, be one, three, or even more.

As an implementation, the fourth support member 121 includes an incubation tray, and the heating member is configured to heat the nucleic acid extraction container 10 on the incubation tray. The lysis zone 1211, the washing zone 1212, the drying zone 1213, and the elution zone 1214 are formed in the incubation tray, and a first transfer station 1215 is further formed in the incubation tray. The incubation tray is configured to convey the nucleic acid extraction containers 10 located in each of the lysis zone 1211, the washing zone 1212, the drying zone 1213 and the elution zone 1214 to the first transfer station 1215. The transfer assembly 130 is configured to convey the nucleic acid extraction container 10 located at the first transfer station 1215 to the first functional assembly 110. In this embodiment, the transfer assembly 130 performs the pickup and placement on the nucleic acid extraction container 10 on the fourth support member 121 at only one transfer station (i.e., the first transfer station 1215), facilitating simplification of the movement trajectory and structural complexity of the transfer assembly 130.

As an implementation, the fourth support member 121 is disk-shaped. A plurality of accommodating holes arranged along a horizontal circumferential direction are formed on the fourth support member 121. Each accommodating hole is configured to accommodate one nucleic acid extraction container 10. The first functional assembly 110 includes a first support member 111, a second support member 112, a third support member 113, and at least three connecting plates. The at least three connecting plates are sequentially spaced apart from one another. The first support member 111, the second support member 112, and the third support member 113 are respectively mounted on corresponding connecting plates. The first support member 111 is configured to support the nucleic acid extraction container 10 for performing a first liquid aspiration operation and a first liquid dispensing operation for dispensing a cleaning liquid. The second support member 112 is configured to support the nucleic acid extraction container 10 for performing a second liquid aspiration operation. The third support member 113 is configured to support the nucleic acid extraction container 10 for performing a second liquid dispensing operation for dispensing an elution reagent.

As an implementation, the sample analyzer 1 further includes a frame assembly. The nucleic acid extraction device 100 is mounted on the frame assembly. The fourth support member 121 is movably mounted on the frame assembly and configured to support the nucleic acid extraction container 10 for performing an incubation operation, and at least one first transfer station 1215 and a plurality of accommodating holes are formed on the fourth support member 121. Each accommodating hole is configured to accommodate one nucleic acid extraction container 10. The first driving member 122 is configured to drive the fourth support member 121 to move relative to the frame assembly, such that the fourth support member 121 drives each of the plurality of nucleic acid extraction containers 10 to move to the first transfer station 1215. The first support member 111 is fixed to the frame assembly. The transfer assembly 130 is configured to at least transfer the nucleic acid extraction container 10 between the first transfer station 1215 and the first support member 111. The first pipetting member 118 is configured to perform a first liquid aspiration operation on the nucleic acid extraction container 10 located on the first support member 111. In this embodiment, the support members of the first functional assembly 110 are stationary relative to the frame assembly, while the support member of the second functional assembly 120 is movable relative to the frame assembly.

As an implementation, the first support member 111, the second support member 112, and the third support member 113 are mounted on the frame assembly in a manner stationary relative to the frame assembly, so as to support the nucleic acid extraction container 10 for performing a first liquid aspiration operation, a second liquid aspiration operation, and a second liquid dispensing operation, respectively. That is, the second support member 112 and the third support member 113 are also mounted on the frame assembly in a stationary manner relative to the frame assembly to support the nucleic acid extraction container 10. The second pipetting member 114 is configured to perform a second liquid aspiration operation on the nucleic acid extraction container 10 located on the second support member 112 and having been subjected to the first liquid aspiration operation. The third pipetting member 117 is configured to perform a second liquid dispensing operation for dispensing the elution reagent into the nucleic acid extraction container 10 located on the third support member 113 and having been subjected to the second liquid aspiration operation. The transfer assembly 130 is further configured to: transfer the nucleic acid extraction container 10 between the first transfer station 1215 and the second support member 112, and transfer the nucleic acid extraction container 10 between the first transfer station 1215 and the third support member 113.

As an implementation, the first support member 111, the second support member 112, and the third support member 113 are sequentially disposed side-by-side in a horizontal linear direction. The transfer assembly 130 includes a single first gripping member and a single second driving member. The first gripping member is configured to grip and release the nucleic acid extraction container 10. The second driving member is configured to drive the first gripping member to perform a three-dimensional linear movement, such that the first gripping member is moved to the first transfer station 1215, the first support member 111, the second support member 112, and the third support member 113, respectively, to grip and release the nucleic acid extraction container 10.

As an implementation, the sample analyzer 1 further includes a controller 600. The controller 600 is configured to: control the transfer assembly 130 to convey the nucleic acid extraction container 10 subjected to the incubation operation in the lysis zone 1211 to the first support member 111; control the first pipetting member 118 to perform the first liquid aspiration operation on the liquid in the nucleic acid extraction container 10 in the first support member 111, and control the liquid dispensing member to perform the first liquid dispensing operation on the nucleic acid extraction container 10 in the first support member 111; control the transfer assembly 130 to transfer the nucleic acid extraction container 10 subjected to the first liquid dispensing operation to the washing zone 1212 to perform the incubation operation; control the transfer assembly 130 to transfer the nucleic acid extraction container 10 subjected to the incubation operation in the washing zone 1212 to the second support member 112; control the second pipetting member 114 to perform the second liquid aspiration operation on the liquid in the nucleic acid extraction container 10 in the second support member 112; control the transfer assembly 130 to transfer the nucleic acid extraction container 10 subjected to the second liquid aspiration operation to the drying zone 1213 to perform a drying operation on the nucleic acid extraction container 10; control the transfer assembly 130 to transfer the nucleic acid extraction container 10 subjected to the drying operation in the drying zone 1213 to the third support member 113; control the third pipetting member 117 to perform the second liquid dispensing operation on the nucleic acid extraction container 10 on the third support member 113; and control the transfer assembly 130 to transfer the nucleic acid extraction container 10 subjected to the second liquid dispensing operation to the elution zone 1214 to perform an elution operation.

As an implementation, the nucleic acid extraction method includes the following steps: a lysis incubation step, a first liquid aspiration and dispensing step, a first washing incubation step, a second liquid aspiration and dispensing step, a second washing incubation step, a dedicated liquid aspiration step, a drying step, an elution reagent dispensing step, and an elution incubation step. The lysis incubation step mainly includes performing lysis incubation on the liquid in the nucleic acid extraction container 10 in the lysis zone 1211. In particular, a lysis system may be constructed in another device in a preceding step, for example, by adding a lysis reagent and a sample into the nucleic acid extraction container 10, and transferring the nucleic acid extraction container 10 added with the lysis reagent and the sample to the accommodating hole of the lysis zone 1211, where the heating member heats the sample to be lysed in the lysis zone 1211. Upon completion of the lysis incubation step, the nucleic acid extraction container 10 is transferred from the second functional assembly 120 to the first functional assembly 110 to undergo the first liquid aspiration and dispensing step, so as to assist a subsequent washing stage. The first liquid aspiration and dispensing step includes: magnetically attracting magnetic complexes in the nucleic acid extraction container 10; after the magnetic complexes are attracted, aspirating waste liquid from the nucleic acid extraction container 10 and discharging the waste liquid; and dispensing a cleaning liquid after the waste liquid is aspirated. Upon completion of the first liquid aspiration and dispensing step, the nucleic acid extraction container 10 is transferred from the first functional assembly 110 to the second functional assembly 120 to perform the following first washing incubation step: performing washing incubation on the liquid in the nucleic acid extraction container 10 in the first washing zone 1212. Upon completion of the first washing incubation step in the first washing zone 1212, the nucleic acid extraction container 10 is transferred from the second functional assembly 120 to the first functional assembly 110 to undergo the second liquid aspiration and dispensing step. The second liquid aspiration and dispensing step is similar to the first liquid aspiration and dispensing step and is not described in detail herein. Upon completion of the second liquid aspiration and dispensing step, the nucleic acid extraction container 10 is transferred from the first functional assembly 110 to the second functional assembly 120 to undergo the second washing incubation step. The second washing incubation step is similar to the first washing incubation step and is not described in detail herein. Upon completion of the second washing incubation step, the nucleic acid extraction container 10 is transferred from the second functional assembly 120 to the first functional assembly 110 to undergo the dedicated liquid aspiration step. The dedicated liquid aspiration step includes: magnetically attracting the magnetic complexes in the nucleic acid extraction container 10; and after the magnetic beads are attracted, aspirating and discharging waste liquid from the nucleic acid extraction container 10. Upon completion of the dedicated liquid aspiration step, the nucleic acid extraction container 10 is transferred from the first functional assembly 110 to the second functional assembly 120 to undergo the drying step. The drying step includes drying the liquid in the nucleic acid extraction container 10 in the drying zone 1213. Upon completion of the drying step, the nucleic acid extraction container 10 is transferred from the second functional assembly 120 to the first functional assembly 110 to undergo the elution reagent dispensing step. The elution reagent dispensing step includes: dispensing the elution reagent into the reaction well of the nucleic acid extraction container 10. Upon completion of the elution reagent dispensing step, the nucleic acid extraction container 10 is transferred from the first functional assembly 110 to the second functional assembly 120 to undergo the elution incubation step. The elution incubation step includes: performing elution incubation on the liquid in the nucleic acid extraction container 10 in the elution zone 1214.

As an implementation, a bottom of the accommodating hole in the elution zone 1214 is positioned higher than a bottom of the accommodating hole in the lysis zone 1211, higher than a bottom of the accommodating hole in the washing zone 1212, and higher than a bottom of the accommodating hole in the drying zone 1213. Since the volume of the liquid in the nucleic acid extraction container 10 during the lysis and washing stages is greater than the volume of the liquid in the nucleic acid extraction container 10 during the elution stage, in this embodiment, the height position of the nucleic acid extraction container 10 in the elution zone 1214 is set to be higher than those in the lysis zone 1211, the washing zone 1212, and the drying zone 1213, such that the magnetic complexes in the nucleic acid extraction container 10 in the elution zone 1214 can be concentrated as much as possible at the bottom of the nucleic acid extraction container 10, avoiding nucleic acid loss.

As an implementation, the bottom of the accommodating hole in the lysis zone 1211 is positioned at the same height position as the bottom of the accommodating hole in the washing zone 1212 and the bottom of the accommodating hole in the drying zone 1213. The height position of the nucleic acid extraction container 10 in the lysis zone 1211 is the same as those in the washing zone 1212 and the drying zone 1213. Certainly, in practical applications, the respective height positions of the nucleic acid extraction container 10 in at least two of the lysis zone 1211, the washing zone 1212, and the drying zone 1213 may be set to be different.

As an implementation, the second functional assembly 120 further includes a first driving member 122 configured to drive the fourth support member 121 to move such that the fourth support member 121 drives the nucleic acid extraction container 10 to move. The arrangement of the first driving member 122 may drive the nucleic acid extraction containers 10 located in each of the lysis zone 1211, the washing zone 1212, the drying zone 1213, and the elution zone 1214 to be conveyed to the first transfer station 1215, thereby enabling the transfer assembly 130 to transfer the nucleic acid extraction container 10 between the first transfer station 1215 and the first functional assembly 110.

As an implementation, the second functional assembly 120 further includes a mixing member 140 configured to mix the liquid containing at least the sample and the reagent in the nucleic acid extraction container 10 located on the fourth support member 121. In this way, the sufficient mixing of the sample and the reagent is facilitated, thereby facilitating efficient nucleic acid capture, cleaning, and elution.

As an implementation, the mixing member 140 includes a plurality of magnets. The plurality of magnets are alternately arranged on opposite sides of a movement trajectory of the nucleic acid extraction container 10 in a horizontal direction. The magnets may generate a magnetic field, which may generate a magnetic force on the magnetic beads and the magnetic complexes in the nucleic acid extraction container 10. In this embodiment, mixing the liquid in the nucleic acid extraction container 10 using the magnets may prevent the mixing member 140 from directly contacting the liquid in the nucleic acid extraction container 10, avoiding contamination of the mixing member 140.

As an implementation, the mixing member 140 includes at least a first magnet array 141 and a second magnet array 142 both extending horizontally, with each magnet array including a plurality of magnets spaced apart and mounted on a magnet bracket 143. The fourth support member 121 includes a container receiving portion for placement of the nucleic acid extraction container 10. A plurality of accommodating holes for placement of the nucleic acid extraction container 10 are formed in the container receiving portion. The first magnet array 141 includes a plurality of first magnets 1411 disposed in an array. The second magnet array 142 includes a plurality of second magnets 1421 arranged in an array.

As an implementation, the first magnet array 141 and the second magnet array 142 are parallel to each other in a horizontal extending direction R. The first magnet array 141 and the second magnet array 142 being parallel to each other in a horizontal extending direction R means that the individual magnets in the first magnet array 141 and the second magnet array 142 are consecutively arranged in the horizontal extending direction R. In particular, a line connecting the centers of the magnets in the first magnet array 141 extends in the horizontal extending direction R, and a line connecting the centers of the magnets in the second magnet array 142 extends in the horizontal extending direction R, while the magnets may be arranged obliquely relative to the horizontal extending direction R.

As an implementation, the fourth support member 121 and the mixing member 140 are disposed with respect to each other such that when the nucleic acid extraction container 10 is received in the container receiving portion, at least a bottom of the nucleic acid extraction container 10 is located between the first magnet array 141 and the second magnet array 142. That is, the first magnet array 141 and the second magnet array 142 are arranged on opposite sides of the movement trajectory of the nucleic acid extraction container 10 in the horizontal direction. The fourth support member 121 and the mixing member 140 can move horizontally relative to each other such that the mixing member 140 can mix the liquid containing at least the sample and the magnetic beads in the nucleic acid extraction container 10 received in the container receiving portion.

As an implementation, the fourth support member 121 and the mixing member 140 are further disposed with respect to each other such that when the fourth support member 121 and the mixing member 140 move horizontally relative to each other, at least two magnets in the mixing member 140 have different heights relative to the bottom of the nucleic acid extraction container 10 received in the container receiving portion.

Since the fourth support member 121 and the mixing member 140 can move horizontally relative to each other, the fourth support member 121 can drive the nucleic acid extraction container 10 received in the container receiving portion and located between the first magnet array 141 and the second magnet array 142 to move horizontally relative to the first magnet array 141 and the second magnet array 142, thereby generating a magnetic field that varies in the horizontal direction for the nucleic acid extraction container 10 received in the container receiving portion. In addition, when the fourth support member 121 and the mixing member 140 move horizontally relative to each other, at least two magnets in the mixing member 140 are spaced apart from the bottom of the nucleic acid extraction container 10 received in the container receiving portion by different distances in the vertical direction, thereby generating a magnetic field that varies in the vertical direction for the nucleic acid extraction container 10 received in the container receiving portion. By generating the magnetic field that varies in the horizontal direction and the vertical direction, the magnetic beads in the nucleic acid extraction container 10 placed in the varying magnetic field are spatially distributed as much as possible throughout the entire liquid in the nucleic acid extraction container 10, or the movement trajectories of the magnetic beads over time are distributed throughout the entire liquid in the nucleic acid extraction container 10. In particular, the magnetic beads in the nucleic acid extraction container 10 placed in the varying magnetic field can perform a three-dimensional movement (e.g., in circumferential and axial directions), thereby achieving sufficient mixing of the magnetic beads.

When the nucleic acid extraction device 100 is in a working state, the relative horizontal movement between the fourth support member 121 and the mixing member 140 is continuous and uninterrupted.

It is to be noted that, with respect to directional descriptions, the embodiments of the disclosure are described based on the nucleic acid extraction device 100 being in a normal working state. In this normal working state, the nucleic acid extraction container 10 is vertically placed in the container receiving portion of the fourth support member 121, and the axial direction of the nucleic acid extraction container 10 corresponds to the vertical direction.

As an implementation, to enable at least two magnets to have different heights relative to the bottom of the nucleic acid extraction container 10 received in the container receiving portion when the fourth support member 121 and the mixing member 140 move horizontally relative to each other, it may be specified that when the fourth support member 121 and the mixing member 140 move horizontally relative to each other, the at least two magnets in the mixing member 140 can move relative to the fourth support member 121 in the vertical direction. However, this implementation requires an additional mechanism for enabling vertical relative movement between the fourth support member 121 and the mixing member 140, increasing the complexity and cost of the sample analyzer 1.

As another simpler implementation, at least two magnets in the mixing member 140 have different heights relative to the fourth support member 121, that is, the at least two magnets in the mixing member 140 are spaced apart from the fourth support member 121 by different vertical distances. Preferably, no relative movement exists between the mixing member 140 and the fourth support member 121 in the vertical direction. In particular, they cannot move relative to each other in the vertical direction. It can thereby be easily achieved that, when the fourth support member 121 and the mixing member 140 move horizontally relative to each other, at least two magnets have different heights relative to the bottom of the nucleic acid extraction container 10 received in the container receiving portion.

As an implementation, the at least two magnets in the mixing member 140 are mounted on the magnet bracket 143 at different mounting heights H, that is, the at least two magnets in the mixing member 140 are fixed on the magnet bracket 143 at different heights, such that the at least two magnets in the mixing member 140 have different heights relative to the fourth support member 121.

It may be understood that the mounting height H may be a vertical distance between a bottom surface of the magnet and a bottom surface of the magnet bracket 143 when the magnet is mounted on the magnet bracket 143.

As an implementation, at least two magnets in the first magnet array 141 are mounted on the magnet bracket 143 at different mounting heights H. Alternatively or additionally, at least two magnets in the second magnet array 142 are mounted on the magnet bracket 143 at different mounting heights H.

As an implementation, the magnet bracket 143 may be an integrally manufactured bracket, on which the magnets in the first magnet array 141 and the magnets in the second magnet array 142 are both mounted.

As an implementation, the magnet bracket 143 may include a first bracket and a second bracket, with the magnets in the first magnet array 141 being mounted on the first bracket, and the magnets in the second magnet array 142 being mounted on the second bracket.

As an implementation, the first magnet array 141 includes at least one first magnet 1411 unit and at least one second magnet 1421 unit, with each magnet unit including one magnet or a plurality of consecutively arranged magnets. The magnets in the first magnet 1411 unit (also referred to as first lower-layer magnets) are mounted on the magnet bracket 143 at a first height, and the magnets in the second magnet 1421 unit (also referred to as first upper-layer magnets) are mounted on the magnet bracket 143 at a second height greater than the first height. That is, the first magnet array 141 includes at least two layers of magnets at different heights.

As an implementation, the first magnet array 141 includes two first magnet 1411 units and one second magnet 1421 unit, with each magnet unit including two magnets. Certainly, in practical applications, the arrangement of the first magnets 1411 is not limited thereto.

As an implementation, the second magnet array 142 includes at least one third magnet unit and at least one fourth magnet unit, with each magnet unit including one magnet or a plurality of consecutively arranged magnets. The magnets in the third magnet unit (also referred to as second lower-layer magnets) are mounted on the magnet bracket 143 at a third height, and the magnets in the fourth magnet unit (also referred to as second upper-layer magnets) are mounted on the magnet bracket 143 at a fourth height greater than the third height. That is, the second magnet array 142 includes at least two layers of magnets at different heights.

As an implementation, the first magnet array 141 includes two first magnet 1411 units and one second magnet 1421 unit, with each magnet unit including two magnets. The second magnet array 142 includes two third magnet units and one fourth magnet unit, with each magnet unit including two magnets.

As an implementation, the first height is equal to the third height. Alternatively or additionally, the second height is equal to the fourth height.

As an implementation, the first height may be different from the third height, and the second height may be different from the fourth height.

As an implementation, the first magnet 1411 units and the second magnet 1421 unit are alternately disposed in the horizontal extending direction R, and in particular are sequentially alternately disposed to form the first magnet array 141. In this embodiment, the first magnet array 141 includes two layers of magnets at different heights.

As an implementation, the third magnet units and the fourth magnet unit are alternately disposed in the horizontal extending direction R, and in particular are sequentially alternately disposed to form the second magnet array 142. In this embodiment, the second magnet array 142 includes two layers of magnets at different heights.

As an implementation, the first magnet array 141 may further include at least one fifth magnet unit. The fifth magnet unit includes one magnet or a plurality of consecutively arranged magnets. The magnets in the fifth magnet unit are mounted on the magnet bracket 143 at a fifth height greater than the second height. Optionally, the second magnet array 142 may further include at least one sixth magnet unit. The sixth magnet unit includes one magnet or a plurality of consecutively arranged magnets. The magnets in the sixth magnet unit are mounted on the magnet bracket 143 at a sixth height greater than the fourth height.

As an implementation, the fifth height is equal to the sixth height. In other embodiments, the fifth height may be different from the sixth height.

As an implementation, the first magnet 1411 units, the second magnet 1421 unit, and the fifth magnet unit are alternately disposed in the horizontal extending direction R, and in particular are sequentially alternately disposed to form the first magnet array 141. In this embodiment, the first magnet array 141 includes three layers of magnets at different heights.

As an implementation, the third magnet units, the fourth magnet unit, and the sixth magnet unit are alternately disposed in the horizontal extending direction R, and in particular are sequentially alternately disposed to form the second magnet array 142. In this embodiment, the second magnet array 142 includes three layers of magnets at different heights.

In the above solution, the first magnet array 141 and the second magnet array 142 are respectively formed by alternately disposing magnet units at different heights. When the fourth support member 121 and the mixing member 140 move horizontally relative to each other, the mixing member 140 can generate a magnetic field that uniformly and alternately varies in both the horizontal direction and the vertical direction, such that the magnetic beads in the nucleic acid extraction container 10 placed in the uniformly and alternately varying magnetic field are spatially distributed as uniformly as possible throughout the liquid in the nucleic acid extraction container 10, or the movement trajectories of the magnetic beads over time are uniformly distributed throughout the liquid in the nucleic acid extraction container 10. In particular, the magnetic beads in the nucleic acid extraction container 10 placed in the varying magnetic field can move uniformly in circumferential and axial directions, thereby achieving uniform and sufficient mixing of the magnetic beads.

As an implementation, each magnet unit includes two magnets, but the disclosure is not limited thereto. For example, each magnet unit may include only one magnet, or may include three, four, or more magnets, which will not be specifically limited.

As an implementation, magnet units at different heights may include different numbers of magnets, which will not be specifically limited.

As an implementation, the extending direction R of the first magnet array 141 may be a circumferential direction or a linear direction.

As an implementation, the number of magnets in the first magnet array 141 and the number of magnets in the second magnet array 142 may be equal or different.

As an implementation, the magnets in the first magnet array 141 and the second magnet array 142 may all be magnets of the same specification. It is to be noted that the magnets in the first magnet array 141 and the second magnet array 142 may be configured as magnets of different specifications according to requirements, for example, having different magnetic field strengths, different quantities, and different dimensions, so as to adapt to mixing in different scenarios.

As an implementation, adjacent magnets in the first magnet array 141 and the second magnet array 142 are disposed in a staggered manner in the horizontal extending direction R. That is, the line connecting the centers of adjacent magnets in the first magnet array 141 and the second magnet array 142 is not perpendicular to the extending direction R. By arranging the magnets in the first magnet array 141 and the second magnet array 142 in a staggered manner along the extending direction R, when the nucleic acid extraction container 10 approaches the magnets, the magnetic field strength and gradient generated here reach the maximum values, thereby rapidly driving the movement of the magnetic beads, disturbing the fluid, and promoting spatial mass exchange within the fluid. When the nucleic acid extraction container 10 is positioned between adjacent magnets, the magnetic field gradient generated here decreases sharply, causing the magnetic beads to experience weak forces and to rapidly disperse throughout the spatial flow field under the influence of flow field motion, thereby achieving thorough contact between the magnetic beads and the nucleic acids in the fluid.

As an implementation, the magnets in the first magnet array 141 are spaced apart by the same distance in the horizontal extending direction R. That is, adjacent magnets in the first magnet array 141 have the same spacing in the horizontal extending direction R.

Alternatively or additionally, the magnets in the second magnet array 142 are spaced apart by the same distance in the horizontally extending direction R. That is, adjacent magnets in the second magnet array 142 have the same spacing in the horizontal extending direction R.

As an implementation, the magnets in the first magnet array 141 and the magnets in the second magnet array 142 are both arranged along a circumferential direction as the extending direction R, and the first magnet array 141 is concentrically disposed with the second magnet array 142. In particular, a plurality of first magnets 1411 are arranged in an annular array, a plurality of second magnets 1421 are arranged in an annular array, and the plurality of second magnets 1421 are arranged on the outer periphery of the plurality of first magnets 1411. In this embodiment, the extending direction R of the magnets is a circumferential direction. In other embodiments, the extending direction R of the magnets is a linear direction, and the magnets in the first magnet array 141 and the magnets in the second magnet array 142 are both arranged in a linear direction as the extending direction R.

As an implementation, the magnet bracket 143 and the fourth support member 121 may be configured as a disk, in which case each magnet array is a circular array. In another example, the magnet bracket 143 and the fourth support member 121 may be configured as a sector-shaped disk, in which case each magnet array is an arc-shaped array.

As an implementation, the magnets in the first magnet array 141 have a first magnetic pole facing the fourth support member 121, and the magnets in the second magnet array 142 have a second magnetic pole facing the fourth support member 121. The first magnetic pole has a polarity opposite to that of the second magnetic pole.

In other embodiments, same-side magnetic poles of adjacent magnets in the first magnet array 141 have opposite polarities, and/or same-side magnetic poles of adjacent magnets in the second magnet array 142 have opposite polarities.

Next, some implementations for driving the mixing member 140 to move horizontally relative to the fourth support member 121 are described.

As an implementation, the second functional assembly 120 further includes a third driving member, and the mixing member 140 includes a movable magnet bracket 143 and a plurality of magnets fixed to the magnet bracket 143. The third driving member is configured to drive the magnet bracket 143 to move, so as to drive the plurality of magnets fixed to the magnet bracket 143 to move relative to the nucleic acid extraction container 10 on the fourth support member 121.

As an implementation, the third driving member is configured to drive the magnet bracket 143 to move in the extending direction R, such that the mixing member 140 moves relative to the fourth support member 121 in the extending direction R, thereby driving movement of the magnetic beads in the nucleic acid extraction container 10 located between the first magnet array 141 and the second magnet array 142.

In particular, when the third driving member drives the magnet bracket 143 to move, the magnetic beads in the nucleic acid extraction container 10 may perform a cyclic movement along the circumferential and axial directions of the nucleic acid extraction container 10.

For example, when the third driving member drives the magnet bracket 143 to move in the extending direction R, the nucleic acid extraction container 10 placed on the fourth support member 121 may sequentially pass the magnets in the first magnet array 141 and the second magnet array 142 in the extending direction R at least once, preferably a plurality of times, thereby placing the nucleic acid extraction container 10 in a varying, preferably alternately varying, magnetic field.

For another example, when the third driving member drives the magnet bracket 143 to move in the extending direction R, the nucleic acid extraction container 10 placed on the fourth support member 121 moves back and forth in the extending direction R across at least part of the magnets in the first magnet array 141 and the second magnet array 142, thereby placing the nucleic acid extraction container 10 in a varying, preferably alternately varying, magnetic field.

As an implementation, the fourth support member 121 is configured as a rotatable first disk, and the magnet bracket 143 is configured as a rotatable second disk arranged concentrically with the first disk.

Further preferably, the second disk is configured to be capable of rotating both synchronously with the first disk and independently of the first disk. In particular, the first disk can drive the second disk to rotate together, such that the rotation of the first disk does not affect the relative horizontal movement between the first disk and the second disk caused by the independent rotation of the second disk. That is, a rotating shaft of the second disk is connected to a rotating shaft of the first disk, such that when the first disk rotates, the second disk is driven to rotate synchronously, while the second disk can also rotate independently. In this way, when the position of the nucleic acid extraction container 10 changes due to the rotation of the first disk, the relative movement relationship between the nucleic acid extraction container 10 and the magnetic field may remain substantially unchanged.

As an implementation, the mixing member 140 includes a movable magnet bracket 143 and a plurality of magnets fixed to the magnet bracket 143. The third driving member is configured to drive the magnet bracket 143 to move so as to drive the plurality of magnets fixed to the magnet bracket 143 to move relative to the nucleic acid extraction container 10 on the fourth support member 121, such that the magnetic beads in the nucleic acid extraction container 10 placed on the fourth support member 121 perform a three-dimensional movement in the liquid in the nucleic acid extraction container 10 under the effect of the moving magnets.

As an implementation, the mixing member 140 is configured to generate a magnetic field, and the third driving member is configured to drive relative movement between the fourth support member 121 and the mixing member 140, such that the mixing member 140 generates an alternately varying magnetic field relative to the nucleic acid extraction container 10 on the fourth support member 121, enabling the magnetic beads in the nucleic acid extraction container 10 to perform a three-dimensional movement in the liquid in the nucleic acid extraction container 10 under the effect of the alternately varying magnetic field.

As an implementation, the fourth support member 121 includes at least a lysis zone 1211, and the nucleic acid extraction container 10 undergoes a lysis and capture stage in the lysis zone 1211. During the lysis and capture stage, cells in the sample in the nucleic acid extraction container 10 are lysed to release nucleic acids, and the released nucleic acids are captured by the magnetic beads in the nucleic acid extraction container 10. The mixing member 140 is configured to generate a magnetic field covering at least the lysis zone 1211. The third driving member is configured to drive relative movement between the fourth support member 121 and the mixing member 140, such that the magnetic beads in the nucleic acid extraction container 10 located in the lysis zone 1211 are moved so as to capture the released nucleic acids.

As an implementation, the nucleic acid extraction device 100 is divided into an upper part and a lower part. The upper part of the nucleic acid extraction device 100 is primarily configured to implement functions such as dispensing of the cleaning liquid, magnetic bead separation, waste liquid aspiration and discharge, and dispensing of the elution reagent. The lower part of the nucleic acid extraction device 100 is divided into five zones: a lysis zone 1211, a first washing zone 1212, a second washing zone 1212, a drying zone 1213, and an elution zone 1214, in which functions such as lysis, washing, drying, and elution are respectively implemented. The nucleic acid extraction container 10 may be griped and transferred vertically by the transfer assembly 130 and placed at different stations to perform different functional operations.

As an implementation, the transfer assembly 130 is a manipulator. The functional assemblies in the upper part of the nucleic acid extraction device 100 are divided into different stations to implement corresponding functions. The lower part of the nucleic acid extraction device 100 is divided into different zones, and a distinct function is performed in each zone. The lower part of the nucleic acid extraction device cooperates with the manipulator to grip and transfer the nucleic acid extraction container 10.

As an implementation, the upper part of the nucleic acid extraction device 100 is provided with four stations. Station I is configured to: perform operations of attracting the magnetic beads, aspirating the waste liquid, and dispensing a cleaning liquid on the lysed sample in the nucleic acid extraction container 10. Station II is configured to: perform operations of aspirating the waste liquid and dispensing a cleaning liquid on the sample in the nucleic acid extraction container 10 that has been subjected to cleaning incubation in the first washing zone 1212. Station III is configured to: perform an operation of aspirating the waste liquid on the sample in the nucleic acid extraction container 10 that has been subjected to cleaning incubation in the second washing zone 1212. Station IV is configured to: perform an operation of dispensing an elution reagent on the sample in the nucleic acid extraction container 10 that has been subjected to drying incubation in the drying zone 1213.

As an implementation, the transfer assembly 130 is configured to: grip the nucleic acid extraction container 10 from the lower second functional assembly 120 (i.e., a mixing and incubation module) to the four stations of the upper first functional assembly 110, and to grip the nucleic acid extraction container from the four stations of the upper first functional assembly 110 and place same onto the lower second functional assembly 120 for operation. In an alternative embodiment, the transfer assembly 130 may be configured to transfer the nucleic acid extraction container 10 between different stations of the upper first functional assembly 110 and/or to transfer the nucleic acid extraction container 10 between different zones of the lower second functional assembly 120.

As an implementation, the amplification device 300 is primarily configured to amplify the nucleic acids extracted by the nucleic acid extraction device 100, so as to substantially increase the amount of nucleic acids within a relatively short period of time. The amplification treatment may be performed using methods such as polymerase chain reaction (PCR), loop mediated isothermal amplification (LAMP), isothermal chimeric primer initiated amplification (ICAN), nucleic acid sequence-based amplification (NASBA), strand displacement amplification (SDA), ligase chain reaction (LCR), and rolling circle amplification (RCA).

As an implementation, the test device 400 is primarily configured to test the amplified nucleic acids. For example, the test device 400 is configured to perform fluorescence testing on the amplified nucleic acids. During nucleic acid amplification and real-time fluorescence testing, signals of each nucleic acid amplification cycle are read to obtain a fluorescence amplification curve. The test device 400 obtains a qualitative result of negative or positive according to the fluorescence amplification curve, or obtains, based on a calibration curve, a quantitative analysis result and the concentration of a target substance in the sample under test.

As an implementation, the sample analyzer 1 further includes a container providing device 500. The container providing device 500 is configured to provide the nucleic acid extraction container 10 and the amplification reaction container. The sample container, the nucleic acid extraction container 10, and the amplification reaction container are three different types of containers. The sample container is primarily configured to accommodate a sample collected from a patient, that is, the sample container is primarily configured to provide an accommodating site for the sample. The nucleic acid extraction container 10 is primarily configured to receive the sample for nucleic acid extraction, that is, the nucleic acid extraction container 10 is primarily configured to provide a nucleic acid extraction site for the sample. The amplification reaction container is primarily configured to receive the nucleic acid extract for an amplification reaction, that is, the amplification reaction container is primarily configured to provide an amplification reaction site for the nucleic acid extract. The pipetting device 700 is primarily configured to transfer the nucleic acid extract between the nucleic acid extraction container 10 and the amplification reaction container. The nucleic acid extraction container 10 and the amplification reaction container are two different consumables of the sample analyzer 1, such that the nucleic acid extraction container 10 may be designed into a shape that facilitates nucleic acid extraction according to requirements, and the amplification reaction container may be designed into a shape that facilitates amplification reaction according to requirements. The nucleic acid extraction container 10 and the amplification reaction container are used as consumables. In particular, both the nucleic acid extraction container 10 and the amplification reaction container are disposable containers. A nucleic acid extraction container 10 is discarded and recovered after completing the nucleic acid extraction for a single test item, and an amplification reaction container is discarded and recovered after completing the amplification reaction and testing for a single test item, without requiring cleaning and reuse. In this way, the container cleaning step during the testing process may be omitted, thereby improving the testing efficiency and avoiding the problem of cross-contamination caused by insufficient cleaning, which otherwise affects the accuracy of sample test results. For each sample tested in the sample analyzer 1, consumption of at least one nucleic acid extraction container 10 and at least one amplification reaction container is required. In this embodiment, a second consumable supply device provides the nucleic acid extraction container 10, and a first consumable supply device provides the amplification reaction container. In this way, continuous testing requirements for batch samples may be satisfied, thereby avoiding the situations where the absence of the nucleic acid extraction container 10 or the reaction container affects sample testing efficiency.

As an implementation, the container providing device 500 includes a first consumable supply device and a second consumable supply device. The first consumable supply device is configured to at least provide the amplification reaction container. The second consumable supply device is configured to at least provide the nucleic acid extraction container 10. The sample distribution device 200 is configured to aspirate at least part of a sample from a sample container and dispense same into the nucleic acid extraction container 10 provided by the second consumable supply device. The pipetting device 700 is configured to aspirate at least part of the nucleic acid extract from the nucleic acid extraction container 10 and dispense same into the amplification reaction container provided by the first consumable supply device.

As an implementation, the first consumable supply device is configured to store amplification reaction containers, that is, the first consumable supply device may buffer a number of amplification reaction containers to satisfy the supply requirements for batch amplification reaction containers.

As an implementation, the first consumable supply device is further configured to allow an operator or an operating robot to place the amplification reaction container, enabling loading of the amplification reaction container, that is, a storage site and a loading site of the amplification reaction container in the sample analyzer 1 are at the same position. Certainly, in practical applications, as an alternative embodiment, the storage site and the loading site of the amplification reaction container in the sample analyzer 1 may be located at two different positions.

As an implementation, the second consumable supply device is configured to store nucleic acid extraction containers 10, that is, the second consumable supply device may buffer a number of nucleic acid extraction containers 10 to satisfy the supply requirements for batch nucleic acid extraction containers 10.

As an implementation, the second consumable supply device is further configured to allow the operator or the operating robot to place the nucleic acid extraction container 10, enabling loading of the nucleic acid extraction container 10, that is, a storage site and a loading site of the nucleic acid extraction container 10 in the sample analyzer 1 are at the same position. Certainly, in practical applications, as an alternative embodiment, the storage site and the loading site of the nucleic acid extraction container 10 in the sample analyzer 1 may be located at two different positions.

As an implementation, the second consumable supply device is disposed below or above the first consumable supply device, that is, the first consumable supply device and the second consumable supply device are disposed in the vertical direction. The first consumable supply device and the second consumable supply device are arranged in separate upper and lower levels. This facilitates the arrangement of a consumable supply device with a relatively large footprint in one level, thereby enabling the sample analyzer 1 to buffer a larger number of consumables while reducing the dimension of the sample analyzer 1 in the horizontal direction. As a result, the footprint of the sample analyzer 1 is effectively reduced, thereby improving space utilization in a laboratory where the sample analyzer 1 is located. Certainly, in practical applications, as an alternative embodiment, the first consumable supply device and the second consumable supply device may be arranged in the horizontal direction.

As an implementation, the sample analyzer 1 further includes a conveying device 903. The conveying device 903 is configured to at least convey the nucleic acid extraction container 10 provided by the container providing device 500 to the nucleic acid extraction device 100, and to convey the amplification reaction container provided by the container providing device 500 to the amplification device 300.

As an implementation, the conveying device 903 includes a first conveying mechanism and a second conveying mechanism. The first conveying mechanism is configured to convey the nucleic acid extraction container 10 provided by the second consumable supply device to the nucleic acid extraction device 100, and the second conveying mechanism is configured to convey the amplification reaction container provided by the first consumable supply device to the amplification device 300.

As an implementation, the sample analyzer 1 further includes a sample storage device 800 configured to at least store a sample container accommodating a sample. The sample distribution device 200 is configured to aspirate at least part of the sample from the sample container provided by the sample storage device 800 and dispense same into the nucleic acid extraction container 10 provided by the second consumable supply device. The sample storage device 800 may store a number of sample containers to satisfy the continuous testing requirements for batch samples.

As an implementation, the sample storage device 800 is further configured to allow the operator or the operating robot to place the sample container, enabling loading of the sample container, that is, a storage site and a loading site of the sample container in the sample analyzer 1 are at the same position. Certainly, in practical applications, as an alternative embodiment, the storage site and the loading site of the sample container in the sample analyzer 1 may be located at two different positions.

As an implementation, the sample container is provided with an identification code which is associated with at least test item information of the sample in the sample container. The sample analyzer 1 further includes an information acquisition component configured to identify the identification code so as to obtain at least the test item information of the sample.

As an implementation, the sample analyzer 1 further includes a reagent storage compartment 901 and a reagent dispensing device 902. The reagent storage compartment 901 is configured to store a reagent, and the reagent dispensing device 902 is configured to dispense the reagent provided by the reagent storage device into the nucleic acid extraction container 10 and/or the amplification reaction container. In this embodiment, the reagent is dispensed into the nucleic acid extraction container 10 and/or the amplification reaction container using the reagent dispensing device 902. Certainly, in practical applications, as an alternative embodiment, the reagent may be pre-stored in the nucleic acid extraction container 10 and/or the amplification reaction container. In this way, it is also possible that the sample analyzer 1 is provided with neither the reagent storage compartment 901 nor the reagent dispensing device 902.

As an implementation, the reagent storage compartment 901 is configured to store a first-type reagent and a second-type reagent. The first-type reagent is configured to react with the sample to obtain a nucleic acid extract, and the second-type reagent is configured to react with the nucleic acid extract for amplification. The reagent dispensing device 902 is configured to dispense the first-type reagent stored in the reagent storage compartment 901 into the nucleic acid extraction container 10, and to dispense the second-type reagent stored in the reagent storage compartment 901 into the amplification reaction container. The first-type reagent is mainly used for nucleic acid extraction and is also referred to as an extraction reagent. The second-type reagent is mainly used for amplification reaction and is also referred to as an amplification reagent.

As an implementation, the first-type reagent includes a lysis reagent, a magnetic bead reagent, and an elution reagent.

As an implementation, the reagent storage compartment 901 is further configured to allow the operator or the operating robot to place the reagent, enabling loading of the reagent, that is, a storage site and a loading site of the reagent in the sample analyzer 1 are at the same position. Certainly, in practical applications, as an alternative embodiment, the storage site and the loading site of the reagent in the sample analyzer 1 may be located at two different positions.

As an implementation, the reagent storage compartment 901 is configured to place a first-type reagent container and a second-type reagent container. The first-type reagent container is configured to accommodate the first-type reagent, and the second-type reagent container is configured to accommodate the second-type reagent. The reagent dispensing device 902 is configured to aspirate the first-type reagent from the first-type reagent container and dispense same into the nucleic acid extraction container 10, and to aspirate the second-type reagent from the second-type reagent container and dispense same into the amplification reaction container.

As an implementation, the reagent storage compartment 901 includes a first reagent storage assembly and a second reagent storage assembly. The first reagent storage assembly is configured to store the first-type reagent. The reagent dispensing device 902 is configured to aspirate the first-type reagent from the first reagent storage assembly and dispense same into the nucleic acid extraction container 10. The nucleic acid extraction device 100 is configured to perform a nucleic acid extraction operation on the liquid containing at least the sample and the first-type reagent in the nucleic acid extraction container 10 to obtain a nucleic acid extract. The second reagent storage assembly is configured to store the second-type reagent. The reagent dispensing device 902 is configured to aspirate the second-type reagent from the second reagent storage assembly and dispense same into the amplification reaction container. The amplification device 300 is configured to amplify the liquid prepared at least from the nucleic acid extract and the second-type reagent in the amplification reaction container to obtain a test solution.

As an implementation, the sample analyzer 1 is a molecular diagnostic integrated machine for testing RNA or DNA by nucleic acid extraction.

As an implementation, the nucleic acid extraction device 100 is divided into two main parts: an upper part and a lower part. The upper part of the nucleic acid extraction device 100 is primarily configured to implement functions such as dispensing of the cleaning liquid, magnetic attraction, and waste liquid aspiration and discharge. The lower part of the nucleic acid extraction device 100 is primarily configured to implement functions such as incubation and mixing. In this embodiment, key functional modules are designed in a vertical layout, and components having similar functions are designed to be integrated into a functional module. For example, as an implementation, the lower part performs functions such as lysis, incubation, mixing, nucleic acid capture, nucleic acid washing, and nucleic acid elution during nucleic acid extraction. The upper part implements functions such as liquid dispensing, magnetic separation, and waste liquid aspiration and discharge. One or more manipulator functional modules are further provided to grasp the nucleic acid extraction container 10 and transfer same between the upper and lower functional assemblies and various stations, thereby completing the entire nucleic acid extraction function. In this embodiment, all the stations requiring incubation are integrated into one functional assembly, and all the stations requiring liquid aspiration and dispensing are integrated into another functional assembly. The two functional assemblies are arranged one above the other, and the nucleic acid extraction container 10 is transferred upward and downward therebetween. In this way, components having similar functions are integrated into one functional assembly, the horizontal footprint may be reduced, while helping reduce the design and manufacturing difficulty of the nucleic acid extraction device 100.

### Embodiment II:

Referring to FIGS. 1 to 12 and 19, the sample analyzer 1 according to this embodiment mainly differs from that of Embodiment I in the arrangement of the fourth support member 121 of the second functional assembly 120, specifically as follows: In Embodiment I, the fourth support member 121 of the second functional assembly 120 is movable relative to the frame assembly. In this embodiment, however, the support member of the second functional assembly 120 is stationary relative to the frame assembly.

In particular, the sample analyzer 1 according to this embodiment includes a frame assembly. The nucleic acid extraction device 100 is mounted on the frame assembly. The second functional assembly 120 includes a fourth support member 121. The fourth support member 121 is fixed to the frame assembly and configured to support the nucleic acid extraction container 10 for performing an incubation operation, and at least one first transfer station 1215 and a plurality of accommodating holes are formed on the fourth support member 121, with each accommodating hole being configured to accommodate one nucleic acid extraction container 10. The first support member 111 is fixed to the frame assembly. The transfer assembly 130 is configured to at least transfer the nucleic acid extraction container 10 between the first transfer station 1215 and the first support member 111, and is configured to be capable of performing at least a three-dimensional movement. The fourth support member 121 is mounted on the frame assembly in a manner stationary relative to the frame assembly, and is configured to support the nucleic acid extraction container 10 for performing an incubation operation. The first functional assembly 110 includes a first support member 111 and a first pipetting member 118. The first support member 111 is mounted on the frame assembly in a manner stationary relative to the frame assembly and is configured to support the nucleic acid extraction container 10 for performing a first magnetic attraction operation and a first liquid aspiration operation. The first pipetting member 118 is configured to perform the first liquid aspiration operation on the nucleic acid extraction container 10 located on the first support member 111. The transfer assembly 130 is configured to at least transfer the nucleic acid extraction container 10 between the first transfer station 1215 and the first support member 111. In this embodiment, the support members of the first functional assembly 110 and the second functional assembly 120 for supporting the nucleic acid extraction container 10 are fixedly disposed relative to the frame assembly.

As an implementation, at least two first transfer stations 1215 are formed on the second functional assembly 120. The first functional assembly 110 further includes a second support member 112, a second pipetting member 114, a third support member 113, and a third pipetting member 117. The second support member 112 and the third support member 113 are both mounted on the frame assembly in a manner stationary relative to the frame assembly and are configured to support the nucleic acid extraction container 10 for performing a first liquid aspiration operation. The second pipetting member 114 is configured to perform a second liquid aspiration operation on the nucleic acid extraction container 10 located on the second support member 112 and having been subjected to the first liquid aspiration operation. The third pipetting member 117 is configured to perform a second liquid dispensing operation for dispensing an elution reagent into the nucleic acid extraction container 10 located on the third support member 113 and having been subjected to the second liquid aspiration operation. A lysis zone 1211, a washing zone 1212, a drying zone 1213, and an elution zone 1214 are formed on the fourth support member 121. The number of first transfer stations 1215 is equal to the total number of lysis zones 1211, washing zones 1212, drying zones 1213, and elution zones 1214, and each lysis zone 1211, each washing zone 1212, each drying zone 1213, and each elution zone 1214 is correspondingly provided with one first transfer station 1215. The transfer assembly 130 is configured to: transfer the nucleic acid extraction container 10 between the first transfer stations 1215 corresponding to the lysis zone 1211, the washing zone 1212, the drying zone 1213, and the elution zone 1214, and the first support member 111, the second support member 112, and the third support member 113.

As an implementation, the first functional assembly 110 includes two first support members 111, one second support member 112, and one third support member 113. One lysis zone 1211, two washing zones 1212, one drying zone 1213, and one elution zone 1214 are formed on the fourth support member 121. The number of first transfer stations 1215 is five, and each lysis zone 1211, each washing zone 1212, each drying zone 1213, and each elution zone 1214 is correspondingly provided with one first transfer station 1215.

As an implementation, the first support member 111, the second support member 112, and the third support member 113 are sequentially disposed side-by-side in a horizontal linear direction, and the first transfer stations 1215 corresponding to the lysis zone 1211, the washing zone 1212, the drying zone 1213, and the elution zone 1214 are spaced apart and arranged in a horizontal circumferential direction.

As an implementation, the transfer assembly 130 includes a manipulator, which may drive the nucleic acid extraction container 10 to move to the first support member 111, the second support member 112, the third support member 113, and the first transfer stations 1215 corresponding to the lysis zone 1211, the washing zone 1212, the drying zone 1213, and the elution zone 1214.

In this embodiment, the support members of the first functional assembly 110 and the second functional assembly 120 for supporting the nucleic acid extraction container 10 are fixedly disposed relative to the frame assembly. Transfer of the nucleic acid extraction container 10 between the first functional assembly 110 and the second functional assembly 120 is achieved through movement of the transfer assembly 130.

Except for the foregoing, the rest of the sample analyzer 1 according to this embodiment may refer to Embodiment I and will not be described in detail herein.

### Embodiment III:

The sample analyzer 1 according to this embodiment mainly differs from that of Embodiment II in the arrangement of the support members of the first functional assembly 110, specifically as follows: In Embodiment II, the support members of the first functional assembly 110 are disposed side-by-side in a horizontal linear direction. In this embodiment, however, the support members of the first functional assembly 110 are spaced apart in a horizontal circumferential direction.

In particular, in the sample analyzer 1 according to this embodiment, at least two first transfer stations 1215 are sequentially spaced apart in a horizontal circumferential direction. The first support member 111, the second support member 112, and the third support member 113 are sequentially spaced apart in the horizontal circumferential direction. The transfer assembly 130 includes a single first gripping member, a single second driving member, and a single rotation driving member. The first gripping member is configured to grip and release the nucleic acid extraction container 10. The rotation driving member is configured to drive the first gripping member and the second driving member to rotate horizontally, such that the first gripping member is rotated to the same orientation as the first transfer station 1215, the first support member 111, the second support member 112, and the third support member 113, respectively. The second driving member is configured to drive the first gripping member to perform a two-dimensional linear movement, such that the first gripping member is moved to the first transfer station 1215, the first support member 111, the second support member 112, and the third support member 113, respectively, to grip and release the nucleic acid extraction container 10. In this embodiment, the first support member 111, the second support member 112, and the third support member 113 of the upper layer are spaced apart and arranged in the horizontal circumferential direction, and the first transfer stations 1215 of the lower layer corresponding to the lysis zone 1211, the washing zone 1212, the drying zone 1213, and the elution zone 1214 are spaced apart and arranged in the horizontal circumferential direction. The transfer assembly 130 is rotated such that the first gripping member faces each station.

Except for the foregoing, the rest of the sample analyzer 1 according to this embodiment may refer to Embodiment I and Embodiment II and will not be described in detail herein.

### Embodiment IV:

The sample analyzer 1 according to this embodiment mainly differs from that of Embodiment III in the arrangement of the transfer assembly 130, specifically as follows: In Embodiment III, the transfer assembly 130 includes a single first gripping member, a single second driving member, and a single rotation driving member. The first gripping member is aligned with each first transfer station 1215 by means of rotation. In this embodiment, however, the transfer assembly 130 is provided with one first gripping member corresponding to each first transfer station 1215, so that the first gripping member may be aligned with the corresponding first transfer station 1215 without the need to rotate.

In particular, in the sample analyzer 1 according to this embodiment, at least two first transfer stations 1215 are sequentially spaced apart in a horizontal circumferential direction. The first support member 111, the second support member 112, and the third support member 113 are sequentially spaced apart in the horizontal circumferential direction. The transfer assembly 130 includes at least two first gripping members and at least two second driving members. The number of first gripping members and the number of second driving members are equal to the number of first transfer stations 1215. Each first gripping member is configured to grip and release the nucleic acid extraction container 10 at one of the first support member 111, the second support member 112, and the third support member 113, and one corresponding first transfer station 1215. Each second driving member is correspondingly configured to drive one of the first gripping members to perform a two-dimensional linear movement, such that the first gripping member is moved to one of the first support member 111, the second support member 112, and the third support member 113, and to one corresponding first transfer station 1215, for griping and releasing the nucleic acid extraction container 10. In this embodiment, one first gripping member and one second driving member are provided corresponding to each first transfer station 1215, and the first gripping member does not need to rotate.

Except for the foregoing, the rest of the sample analyzer 1 according to this embodiment may refer to Embodiment I to Embodiment III and will not be described in detail herein.

### Embodiment V:

The sample analyzer 1 according to this embodiment mainly differs from that of Embodiment I in the arrangement of the support members of the first functional assembly 110, specifically as follows: In Embodiment I, the support members of the first functional assembly 110 are stationary relative to the frame assembly. In this embodiment, however, the support members of the first functional assembly 110 are movable relative to the frame assembly.

In particular, the sample analyzer 1 according to this embodiment includes a frame assembly. The nucleic acid extraction device 100 is mounted on the frame assembly. The second functional assembly 120 includes a fourth support member 121 and a first driving member 122. The fourth support member 121 is movably mounted on the frame assembly and configured to support the nucleic acid extraction container 10 for performing an incubation operation, and at least one first transfer station 1215 and a plurality of accommodating holes are formed on the fourth support member 121. Each accommodating hole is configured to accommodate one nucleic acid extraction container 10. The first driving member 122 is configured to drive the fourth support member 121 to move relative to the frame assembly, such that the fourth support member 121 drives each of the nucleic acid extraction containers 10 to move to the first transfer station 1215. The first functional assembly 110 includes a first support member 111, a first pipetting member 118, and a second driving member. The first support member 111 is movably mounted on the frame assembly and is configured to support the nucleic acid extraction container 10 for performing a first liquid aspiration operation. The second driving member is configured to drive the first support member 111 to move relative to the frame assembly, such that the first support member 111 drives the nucleic acid extraction container 10 to move to a second transfer station. The first pipetting member 118 is configured to perform the first liquid aspiration operation on the nucleic acid extraction container 10 located on the first support member 111. The transfer assembly 130 is configured to transfer the nucleic acid extraction container 10 between the first transfer station 1215 and the second transfer station.

As an implementation, the first functional assembly 110 further includes a second support member 112, a third support member 113, a fourth driving member, and a fifth driving member. A lysis zone 1211, a washing zone 1212, a drying zone 1213, and an elution zone 1214 are formed on the fourth support member 121. The number of first transfer stations 1215 is one, and the number of second transfer stations is one. The second support member 112 and the third support member 113 are each movably mounted on the frame assembly. The fourth driving member is configured to drive the second support member 112 to move relative to the frame assembly, such that the second support member 112 drives the nucleic acid extraction container 10 to move to the second transfer station. The fifth driving member is configured to drive the third support member 113 to move relative to the frame assembly, such that the third support member 113 drives the nucleic acid extraction container 10 to move to the second transfer station.

As an implementation, the first functional assembly 110 includes two first support members 111, one second support member 112, and one third support member 113. One lysis zone 1211, two washing zones 1212, one drying zone 1213, and one elution zone 1214 are formed on the fourth support member 121.

As an implementation, the first support member 111, the second support member 112, and the third support member 113 are sequentially disposed side-by-side in a horizontal linear direction, and the first transfer stations 1215 corresponding to the lysis zone 1211, the washing zone 1212, the drying zone 1213, and the elution zone 1214 are spaced apart and arranged in a horizontal circumferential direction.

As an implementation, the transfer assembly 130 includes a manipulator, which may drive the nucleic acid extraction container 10 to move to the first transfer station 1215 and the second transfer station.

In this embodiment, the support members of the first functional assembly 110 and the second functional assembly 120 for supporting the nucleic acid extraction container 10 are movably disposed relative to the frame assembly.

Except for the foregoing, the rest of the sample analyzer 1 according to this embodiment may refer to Embodiment I to Embodiment IV and will not be described in detail herein.

### Embodiment VI:

The sample analyzer 1 according to this embodiment mainly differs from that of Embodiment I in the arrangement of the support members of the first functional assembly 110 and the second functional assembly 120, specifically as follows: In Embodiment I, the support members of the first functional assembly 110 are stationary relative to the frame assembly, while the support member of the second functional assembly 120 is movable relative to the frame assembly. In this embodiment, however, the support members of the first functional assembly 110 are movable relative to the frame assembly, while the support member of the second functional assembly 120 is stationary relative to the frame assembly.

In particular, the sample analyzer 1 according to this embodiment includes a frame assembly. The nucleic acid extraction device 100 is mounted on the frame assembly. The second functional assembly 120 includes a fourth support member 121. The fourth support member 121 is mounted on the frame assembly in a manner stationary relative to the frame assembly, and is configured to support the nucleic acid extraction container 10 for performing an incubation operation. The first functional assembly 110 includes a first support member 111, a first pipetting member 118, and a second driving member. The first support member 111 is movably mounted on the frame assembly and is configured to support the nucleic acid extraction container 10 for performing a first liquid aspiration operation. The second driving member is configured to drive the first support member 111 to move relative to the frame assembly, such that the first support member 111 drives the nucleic acid extraction container 10 to move to a second transfer station. The first pipetting member 118 is configured to perform the first liquid aspiration operation on the nucleic acid extraction container 10 located on the first support member 111. The transfer assembly 130 is configured to transfer the nucleic acid extraction container 10 between the fourth support member 121 and the second transfer station. In this embodiment, the support member of the second functional assembly 120 located at the lower position is stationary, while the support members of the first functional assembly 110 located at the upper position are movable.

As an implementation, the first functional assembly 110 further includes a second support member 112, a third support member 113, a fourth driving member, and a fifth driving member. A lysis zone 1211, a washing zone 1212, a drying zone 1213, and an elution zone 1214 are formed on the fourth support member 121. The number of second transfer stations is one. The second support member 112 and the third support member 113 are each movably mounted on the frame assembly. The fourth driving member is configured to drive the second support member 112 to move relative to the frame assembly, such that the second support member 112 drives the nucleic acid extraction container 10 to move to the second transfer station. The fifth driving member is configured to drive the third support member 113 to move relative to the frame assembly, such that the third support member 113 drives the nucleic acid extraction container 10 to move to the second transfer station.

As an implementation, the first functional assembly 110 includes two first support members 111, one second support member 112, and one third support member 113. One lysis zone 1211, two washing zones 1212, one drying zone 1213, and one elution zone 1214 are formed on the fourth support member 121.

As an implementation, the transfer assembly 130 includes a manipulator, which may drive the nucleic acid extraction container 10 to move to the lysis zone 1211, the washing zone 1212, the drying zone 1213, the elution zone 1214, and the second transfer station.

Except for the foregoing, the rest of the sample analyzer 1 according to this embodiment may refer to Embodiment I to Embodiment V and will not be described in detail herein.

### Embodiment VII:

The sample analyzer 1 according to this embodiment mainly differs from that of Embodiment I in the definitions of the functional assemblies in the upper part and the lower part of the nucleic acid extraction device 100, specifically as follows: In Embodiment I, one of the functional assemblies in the upper part and the lower part of the nucleic acid extraction device 100 is configured to at least perform magnetic attraction and a liquid aspiration operation on the liquid in the nucleic acid extraction container 10, and the other is configured to at least incubate the liquid in the nucleic acid extraction container 10. In this embodiment, however, the specific functional operations of the functional assemblies in the upper part and the lower part of the nucleic acid extraction device 100 are not limited, and it is only required that the upper part and the lower part are configured to perform different functional operations or to perform the same functional operation in sequence.

In particular, the sample analyzer 1 according to this embodiment includes a sample distribution device 200, a nucleic acid extraction device 100, a pipetting device 700, an amplification device 300, and a test device 400. The sample distribution device 200 is configured to aspirate a sample from a sample container and dispense at least part of the aspirated sample into the nucleic acid extraction container 10. The nucleic acid extraction device 100 is configured to extract nucleic acids from a liquid containing at least the sample and a reagent in the nucleic acid extraction container 10 to obtain a nucleic acid extract. The pipetting device 700 is configured to transfer the nucleic acid extract from the nucleic acid extraction container 10 to an amplification reaction container. The amplification device 300 is configured to amplify the nucleic acid extract in the amplification reaction container to obtain a test solution. The test device 400 is configured to test the test solution. The nucleic acid extraction device 100 includes a first functional assembly 110, a second functional assembly 120, and a transfer assembly 130. The first functional assembly 110 is disposed above or below the second functional assembly 120. The transfer assembly 130 is configured to transfer the nucleic acid extraction container 10 between the first functional assembly 110 and the second functional assembly 120. The first functional assembly 110 is configured to at least support the nucleic acid extraction container 10 for performing a first functional operation, and the second functional assembly 120 is configured to at least support the nucleic acid extraction container 10 for performing a second functional operation, so as to perform nucleic acid extraction. The first functional operation and the second functional operation are either different functional operations or the same functional operation performed in sequence. In this embodiment, the first functional assembly 110 and the second functional assembly 120 may be configured such that one of the two performs magnetic attraction and a liquid aspiration operation on the liquid in the nucleic acid extraction container 10, while the other incubates the liquid in the nucleic acid extraction container 10. Alternatively, it is not necessarily the case that one of the two is configured to perform magnetic attraction and a liquid aspiration operation on the liquid in the nucleic acid extraction container 10, while the other is configured to incubate the liquid in the nucleic acid extraction container 10. For example, both the first functional assembly 110 and the second functional assembly 120 may assemblies integrating incubation, magnetic attraction, and liquid aspiration functions.

As an implementation, the first functional operation includes at least one of a liquid aspiration operation, a liquid dispensing operation, and a magnetic attraction operation. Components having similar functions are integrated into one functional assembly, facilitating the reduction of the design and manufacturing difficulty.

As an implementation, the second functional operation includes at least one of an incubation operation and a mixing operation. Components having similar functions are integrated into one functional assembly, facilitating the reduction of the design and manufacturing difficulty.

As an implementation, the second functional assembly 120 includes a fourth support member 121 and a mixing member 140. The fourth support member 121 is configured to support the nucleic acid extraction container 10 for performing an incubation operation. The mixing member 140 is configured to mix the liquid containing at least the sample and the reagent in the nucleic acid extraction container 10 located on the fourth support member 121. The mixing member 140 includes at least a first magnet array 141 and a second magnet array 142 both extending horizontally, with each magnet array including a plurality of magnets spaced apart and mounted on a magnet bracket 143. The fourth support member 121 includes a container receiving portion for placement of the nucleic acid extraction container 10. The fourth support member 121 and the mixing member 140 are disposed with respect to each other such that when the nucleic acid extraction container 10 is received in the container receiving portion, at least a bottom of the nucleic acid extraction container 10 is located between the first magnet array 141 and the second magnet array 142. The fourth support member 121 and the mixing member 140 can move horizontally relative to each other such that the mixing member 140 can mix the liquid containing at least the sample and the magnetic beads in the nucleic acid extraction container 10 received in the container receiving portion. The fourth support member 121 and the mixing member 140 are further disposed with respect to each other such that when the fourth support member 121 and the mixing member 140 move horizontally relative to each other, at least two magnets in the mixing member 140 have different heights relative to the bottom of the nucleic acid extraction container 10 received in the container receiving portion.

As an implementation, the mixing member 140 is configured to generate a magnetic field to mix the liquid containing at least the sample and the reagent in the nucleic acid extraction container 10 located on the fourth support member 121.

As an implementation, the second functional assembly 120 further includes a third driving member, and the mixing member 140 includes a movable magnet bracket 143 and a plurality of magnets fixed to the magnet bracket 143. The third driving member is configured to drive the magnet bracket 143 to move, so as to drive the plurality of magnets fixed to the magnet bracket 143 to move relative to the nucleic acid extraction container 10 on the fourth support member 121.

As an implementation, the third driving member is configured to drive the magnet bracket 143, so as to move the plurality of magnets fixed to the magnet bracket 143 relative to the nucleic acid extraction container 10 on the fourth support member 121. This is mainly used to enable magnetic beads in the nucleic acid extraction container 10 placed on the fourth support member 121 to perform a three-dimensional movement in the liquid in the nucleic acid extraction container 10 under the effect of the moving magnets.

As an implementation, the second functional assembly 120 includes a fourth support member 121, a mixing member 140, and a third driving member. The fourth support member 121 is configured to support the nucleic acid extraction container 10 for performing an incubation operation. The mixing member 140 is configured to generate a magnetic field to mix the liquid containing at least the sample and the reagent in the nucleic acid extraction container 10 located on the fourth support member 121. The third driving member is configured to drive relative movement between the fourth support member 121 and the mixing member 140, such that the mixing member 140 generates an alternately varying magnetic field relative to the nucleic acid extraction container 10 on the fourth support member 121, enabling magnetic beads in the nucleic acid extraction container 10 to perform a three-dimensional movement in the liquid in the nucleic acid extraction container 10 under the effect of the alternately varying magnetic field. Alternatively, as an implementation, the second functional assembly 120 includes a fourth support member 121, a mixing member 140, and a third driving member. The fourth support member 121 is configured to support the nucleic acid extraction container 10 for performing an incubation operation. The fourth support member 121 includes at least a lysis zone 1211, and the nucleic acid extraction container 10 undergoes a lysis and capture stage in the lysis zone 1211. During the lysis and capture stage, cells in the sample in the nucleic acid extraction container 10 are lysed to release nucleic acids, and the released nucleic acids are captured by magnetic beads in the nucleic acid extraction container 10. The mixing member 140 is configured to generate a magnetic field covering at least the lysis zone 1211 to mix at least the liquid containing at least the sample and the reagent in the nucleic acid extraction container 10 located in the lysis zone 1211. The third driving member is configured to drive relative movement between the fourth support member 121 and the mixing member 140, such that the magnetic beads in the nucleic acid extraction container 10 located in the lysis zone 1211 are moved so as to capture the released nucleic acids.

In the sample analyzer 1 according to this embodiment, one of the first functional assembly 110 and the second functional assembly 120 of the nucleic acid extraction device 100, which are configured to perform different functional operations or to perform the same functional operation in sequence, is disposed above or below the other, and the nucleic acid extraction container 10 is transferred between the first functional assembly 110 and the second functional assembly 120 via the transfer assembly 130. That is, the first functional assembly 110 and the second functional assembly 120, which are configured to perform different functional operations or to perform the same functional operation in sequence on the liquid in the nucleic acid extraction container 10, are vertically disposed one above the other. The transfer assembly 130 transfers the nucleic acid extraction container 10 to the first functional assembly 110 and the second functional assembly 120. By adopting such a configuration, different functional assemblies of the nucleic acid extraction device 100 may be arranged with full utilization of vertical space, thereby helping reduce the horizontal space occupied by the nucleic acid extraction device 100, facilitating a miniaturization design of the sample analyzer 1, and improving space utilization in a laboratory where the sample analyzer 1 is located.

Except for the foregoing, the rest of the sample analyzer 1 according to this embodiment may refer to Embodiment I to Embodiment VI and will not be described in detail herein.

### Embodiment VIII:

The sample analyzer 1 according to this embodiment mainly differs from that of Embodiment I in the definitions of the upper part and the lower part of the nucleic acid extraction device 100, specifically as follows: In Embodiment I, one of the functional assemblies in the upper part and the lower part of the nucleic acid extraction device 100 is configured to at least perform magnetic attraction and a liquid aspiration operation on the liquid in the nucleic acid extraction container 10, and the other is configured to at least incubate the liquid in the nucleic acid extraction container 10. In this embodiment, however, it is mainly required that the nucleic acid extraction device 100 has two stations disposed one above the other.

In particular, the sample analyzer 1 according to this embodiment includes a sample distribution device 200, a nucleic acid extraction device 100, a pipetting device 700, an amplification device 300, and a test device 400. The sample distribution device 200 is configured to aspirate a sample from a sample container and dispense at least part of the aspirated sample into the nucleic acid extraction container 10. The nucleic acid extraction device 100 is configured to extract nucleic acids from a liquid containing at least the sample and a reagent in the nucleic acid extraction container 10 to obtain a nucleic acid extract. The pipetting device 700 is configured to transfer the nucleic acid extract from the nucleic acid extraction container 10 to an amplification reaction container. The amplification device 300 is configured to amplify the nucleic acid extract in the amplification reaction container to obtain a test solution. The test device 400 is configured to test the test solution. The nucleic acid extraction device 100 has at least two stations, with each station being configured to support the nucleic acid extraction container 10 for performing a processing operation for nucleic acid extraction, at least two of the stations being vertically arranged one above the other, and the processing operation including at least one of an incubation operation, a mixing operation, a magnetic attraction operation, a liquid aspiration operation, and a liquid dispensing operation. In this embodiment, the horizontal footprint of the nucleic acid extraction device 100 may be reduced by disposing at least two stations of the nucleic acid extraction device 100 in the vertical direction.

As an implementation, performing nucleic acid extraction on a liquid containing at least the sample and a reagent by the nucleic acid extraction device 100 includes: a process of performing lysis on the liquid containing at least the sample and the reagent, and performing post-lysis processing.

As an implementation, the nucleic acid extraction device 100 includes a first-layer space and a second-layer space. The first-layer space is disposed above or below the second-layer space. A liquid aspiration-dispensing station, a dedicated liquid aspiration station, and a dedicated liquid dispensing station are disposed in the first-layer space. A lysis incubation station, a cleaning incubation station, a drying incubation station, and an elution incubation station are disposed in the second-layer space. The lysis incubation station is configured to support the nucleic acid extraction container 10 for performing a lysis incubation operation. The cleaning incubation station is configured to support the nucleic acid extraction container 10 for performing a cleaning incubation operation. The drying incubation station is configured to support the nucleic acid extraction container 10 for performing a drying incubation operation. The elution incubation station is configured to support the nucleic acid extraction container 10 for performing an elution incubation operation. The liquid aspiration-dispensing station is configured to support the nucleic acid extraction container 10 for performing a first liquid aspiration operation, and a first liquid dispensing operation for dispensing a cleaning liquid. The dedicated liquid aspiration station is configured to support the nucleic acid extraction container 10 for performing a second liquid aspiration operation. The dedicated liquid dispensing station is configured to support the nucleic acid extraction container 10 for performing a second liquid dispensing operation for dispensing an elution reagent.

In the sample analyzer 1 according to this embodiment, at least two stations in the nucleic acid extraction device 100 are vertically arranged one above the other, with each station being configured to support the nucleic acid extraction container 10 for performing at least one processing operation selected from an incubation operation, a mixing operation, a magnetic attraction operation, a liquid aspiration operation, and a liquid dispensing operation. That is, the at least two stations configured to support the nucleic acid extraction container 10 for performing processing operations are vertically arranged one above the other. By adopting such a configuration, a plurality of stations in the nucleic acid extraction device 100 may be arranged with full utilization of vertical space, thereby helping reduce the horizontal space occupied by the nucleic acid extraction device 100, facilitating a miniaturization design of the sample analyzer 1, and improving space utilization in a laboratory where the sample analyzer 1 is located.

Except for the foregoing, the rest of the sample analyzer 1 according to this embodiment may refer to Embodiment I to Embodiment VII and will not be described in detail herein.

### Embodiment IX:

The sample analyzer 1 according to this embodiment mainly differs from that of Embodiment I in the manner of dividing the functional assemblies in the upper part and the lower part of the nucleic acid extraction device 100, specifically as follows: In Embodiment I, the upper part and the lower part of the nucleic acid extraction device 100 are divided according to functional similarity. The upper part is an integration of structures related to liquid aspiration and dispensing functions, and the lower part is an integration of structures related to incubation and mixing. In this embodiment, the upper part and the lower part of the nucleic acid extraction device 100 are divided according to the sequence of operations. The nucleic acid extraction container 10 first completes incubation and liquid aspiration and dispensing operations simultaneously at one station in the lower part, and is then transferred to one station in the upper part to complete incubation and liquid aspiration and dispensing operations simultaneously.

In particular, the sample analyzer 1 according to this embodiment includes a sample distribution device 200, a nucleic acid extraction device 100, a pipetting device 700, an amplification device 300, and a test device 400. The sample distribution device 200 is configured to aspirate a sample from a sample container and dispense at least part of the aspirated sample into the nucleic acid extraction container 10. The nucleic acid extraction device 100 is configured to perform a nucleic acid extraction operation on a liquid containing at least the sample and a reagent in the nucleic acid extraction container 10 to obtain a nucleic acid extract. The pipetting device 700 is configured to transfer the nucleic acid extract from the nucleic acid extraction container 10 to an amplification reaction container. The amplification device 300 is configured to amplify the nucleic acid extract in the amplification reaction container to obtain a test solution. The test device 400 is configured to test the test solution. The nucleic acid extraction device 100 includes a lysis module, a cleaning module, an elution module, and a transfer assembly 130. At least two of the lysis module, the cleaning module and the elution module are arranged in a vertical direction. The lysis module is configured to support the nucleic acid extraction container 10 for performing a lysis operation. The cleaning module is configured to support the nucleic acid extraction container 10 for performing a cleaning operation. The elution module is configured to support the nucleic acid extraction container 10 for performing an elution operation. The transfer assembly 130 is configured to sequentially transfer the nucleic acid extraction container 10 to the lysis module, the cleaning module, and the elution module.

As an implementation, performing nucleic acid extraction on a liquid containing at least the sample and a reagent by the nucleic acid extraction device 100 includes: a process of performing lysis on the liquid containing at least the sample and the reagent, and performing post-lysis processing.

As an implementation, the nucleic acid extraction device 100 further includes a drying module disposed below or above at least one of the lysis module, the cleaning module, and the elution module. The drying module is configured to support the nucleic acid extraction container 10 for performing a drying operation. The transfer assembly 130 is configured to sequentially transfer the nucleic acid extraction container 10 to the lysis module, the cleaning module, the drying module, and the elution module.

As an implementation, the nucleic acid extraction device 100 may be divided into a plurality of functional modules according to the sequence of the nucleic acid extraction process, and the nucleic acid extraction container 10 is sequentially transferred to different functional modules, without performing any backtracking movement. For example, the cleaning module includes a liquid aspiration and dispensing assembly, and further includes an incubation assembly and a mixing assembly; and the elution module includes a liquid aspiration and dispensing assembly, and further includes an incubation assembly and a mixing assembly.

As an implementation, the nucleic acid extraction device 100 further includes a drying module disposed below or above at least one of the lysis module, the cleaning module, and the elution module. The drying module is configured to support the nucleic acid extraction container 10 for performing a drying operation. The transfer assembly 130 is configured to sequentially transfer the nucleic acid extraction container 10 to the lysis module, the cleaning module, the drying module, and the elution module.

As an implementation, the number of cleaning modules may be one, two, three, or more. When there are two cleaning modules, the transfer assembly 130 is configured to sequentially transfer the nucleic acid extraction container 10 to the lysis module, one of the cleaning modules, the other cleaning module, the drying module, and the elution module.

In Embodiment I, all incubation structures and mixing structures are integrated into a single functional assembly. In this embodiment, all incubation structures may be divided into a plurality of sub-incubation blocks, with each sub-incubation block correspondingly provided with other functional components, such as a liquid aspiration and dispensing component, a temperature control component, a magnetic attraction component, and a mixing component.

In the sample analyzer 1 according to this embodiment, at least two of the lysis module, the cleaning module and the elution module of the nucleic acid extraction device 100 are vertically arranged one above the other. That is, functional modules configured to support the nucleic acid extraction container 10 for performing different functional operations in sequence are vertically arranged one above the other. By adopting such a configuration, a plurality of functional modules in the nucleic acid extraction device 100 may be arranged with full utilization of vertical space, thereby helping reduce the horizontal space occupied by the nucleic acid extraction device 100, facilitating a miniaturization design of the sample analyzer 1, and improving space utilization in a laboratory where the sample analyzer 1 is located.

Except for the foregoing, the rest of the sample analyzer 1 according to this embodiment may refer to Embodiment I to Embodiment VIII and will not be described in detail herein.

The foregoing description merely relates to the preferred embodiments of the disclosure, and is not intended to limit the scope of the patent of the disclosure. All equivalent structural variations made according to the content in the specification and the drawings of the disclosure from the concept of the disclosure, or the direct/indirect applications of the contents in other related technical fields, shall all fall within the scope of patent protection of the disclosure.

## Claims

1. A sample analyzer, comprising:
a sample distribution device configured to aspirate a sample from a sample container and dispense at least part of the aspirated sample into a nucleic acid extraction container;
a nucleic acid extraction device configured to extract nucleic acids from a liquid containing at least the sample and a reagent in the nucleic acid extraction container to obtain a nucleic acid extract, the reagent containing magnetic beads;
a pipetting device configured to transfer the nucleic acid extract from the nucleic acid extraction container to an amplification reaction container;
an amplification device configured to amplify the nucleic acid extract in the amplification reaction container to obtain a test solution; and
a test device configured to test the test solution,
wherein the nucleic acid extraction device comprises a first functional assembly, a second functional assembly, and a transfer assembly, the first functional assembly being disposed above or below the second functional assembly, and the transfer assembly being configured to transfer the nucleic acid extraction container between the first functional assembly and the second functional assembly so as to perform the nucleic acid extraction to obtain the nucleic acid extract; and the first functional assembly being configured to at least perform a magnetic attraction and a liquid aspiration on the liquid in the nucleic acid extraction container, and the second functional assembly being configured to at least incubate the liquid in the nucleic acid extraction container.

2. The sample analyzer of claim 1, wherein the first functional assembly comprises a first support member, a first pipetting member, and a first magnetic attraction member, the first support member being configured to support the nucleic acid extraction container, the first magnetic attraction member being configured to perform a first magnetic attraction on the liquid containing at least the sample and the reagent in the nucleic acid extraction container located on the first support member so as to attract the magnetic beads in the nucleic acid extraction container, and the first pipetting member being configured to perform a first liquid aspiration on the liquid in the nucleic acid extraction container located on the first support member and subjected to the first magnetic attraction by the first magnetic attraction member; and the transfer assembly is configured to transfer the nucleic acid extraction container between the first support member and the second functional assembly, wherein the magnetic attraction comprises the first magnetic attraction, and the liquid aspiration comprises the first liquid aspiration.

3. The sample analyzer of claim 2, wherein the first functional assembly further comprises a liquid dispensing member configured to perform a first liquid dispensing for dispensing a cleaning liquid into the nucleic acid extraction container located on the first support member.

4. The sample analyzer of claim 3, wherein the first functional assembly further comprises a second support member, a second pipetting member, and a second magnetic attraction member, the second support member being configured to support the nucleic acid extraction container, the second magnetic attraction member being configured to perform a second magnetic attraction on a liquid containing at least the cleaning liquid in the nucleic acid extraction container located on the second support member, and the second pipetting member being configured to perform a second liquid aspiration on the liquid in the nucleic acid extraction container located on the second support member and subjected to the second magnetic attraction by the second magnetic attraction member; and the transfer assembly is further configured to transfer the nucleic acid extraction container between the second support member and the second functional assembly, wherein the magnetic attraction further comprises the second magnetic attraction, and the liquid aspiration further comprises the second liquid aspiration.

5. The sample analyzer of claim 4, wherein the first functional assembly further comprises a third support member and a third pipetting member, the third support member being configured to support the nucleic acid extraction container, and the third pipetting member being configured to perform a second liquid dispensing for dispensing an elution reagent into the nucleic acid extraction container located on the third support member; and the transfer assembly is further configured to transfer the nucleic acid extraction container between the third support member and the second functional assembly.

6. The sample analyzer of claim 5, further comprising a first lifting member, wherein the first lifting member is connected to the second magnetic attraction member and is configured to drive the second magnetic attraction member to move up and down relative to the nucleic acid extraction container.

7. The sample analyzer of claim 5, wherein a reaction well, a first tip well, and a second tip well are formed in the nucleic acid extraction container, the reaction well being configured to receive the sample and the reagent for nucleic acid extraction, the first tip well being configured to accommodate a first pipetting tip, and the second tip well being configured to accommodate a second pipetting tip, wherein a capacity of the first pipetting tip is greater than a capacity of the second pipetting tip;
the first pipetting member comprises a first tip connecting component and a first aspiration-discharge drive component, the first tip connecting component being configured to be removably connected to the first pipetting tip so as to perform liquid aspiration by the first pipetting tip under power provided by the first aspiration-discharge drive component; and
the third pipetting member comprises a second tip connecting component and a second aspiration-discharge drive component, the second tip connecting component being configured to be removably connected to the second pipetting tip so as to perform liquid aspiration and liquid dispensing by the second pipetting tip under power provided by the second aspiration-discharge drive component.

8. The sample analyzer of claim 7, wherein a waste liquid well is further formed in the nucleic acid extraction container, and the first pipetting member and the second pipetting member are further configured to dispense liquid aspirated from the reaction well into the waste liquid well.

9. The sample analyzer of claim 3, wherein at least two first support members are provided, with each first support member being correspondingly provided with at least one liquid dispensing member and at least one first pipetting member.

10. The sample analyzer of claim 5, wherein the first support member, the second support member, and the third support member are disposed side-by-side in a horizontal direction.

11. The sample analyzer of claim 5, wherein the second functional assembly comprises a fourth support member and a heating member, the fourth support member being configured to support the nucleic acid extraction container for performing an incubation, and the heating member being configured to heat the liquid containing at least the sample and the reagent in the nucleic acid extraction container located on the fourth support member; and the fourth support member comprising a lysis zone, a washing zone, a drying zone, and an elution zone, wherein an accommodating hole for placement of the nucleic acid extraction container is formed in each of the lysis zone, the washing zone, the drying zone and the elution zone, and the transfer assembly is configured to transfer the nucleic acid extraction container between the lysis zone, the washing zone and the first support member, between the washing zone, the drying zone and the second support member, and between the drying zone, the elution zone and the third support member.

12. The sample analyzer of claim 11, wherein the fourth support member comprises an incubation tray, and the heating member is configured to heat the nucleic acid extraction container on the incubation tray; and the lysis zone, the washing zone, the drying zone, and the elution zone are formed on the incubation tray, and a first transfer station is further formed on the incubation tray, wherein the incubation tray is configured to convey the nucleic acid extraction container located in each of the lysis zone, the washing zone, the drying zone, and the elution zone to the first transfer station, and the transfer assembly is configured to convey the nucleic acid extraction container located at the first transfer station to the first functional assembly.

13. The sample analyzer of claim 11, further comprising a controller configured to:
control the transfer assembly to convey the nucleic acid extraction container subjected to the incubation in the lysis zone to the first support member;
control the first pipetting member to perform the first liquid aspiration on the liquid in the nucleic acid extraction container in the first support member, and control the liquid dispensing member to perform the first liquid dispensing on the nucleic acid extraction container in the first support member;
control the transfer assembly to transfer the nucleic acid extraction container subjected to the first liquid dispensing to the washing zone to perform the incubation;
control the transfer assembly to transfer the nucleic acid extraction container subjected to the incubation in the washing zone to the second support member;
control the second pipetting member to perform the second liquid aspiration on the liquid in the nucleic acid extraction container in the second support member;
control the transfer assembly to transfer the nucleic acid extraction container subjected to the second liquid aspiration to the drying zone to perform a drying on the nucleic acid extraction container;
control the transfer assembly to transfer the nucleic acid extraction container subjected to the drying in the drying zone to the third support member;
control the third pipetting member to perform the second liquid dispensing on the nucleic acid extraction container in the third support member; and
control the transfer assembly to transfer the nucleic acid extraction container subjected to the second liquid dispensing to the elution zone to perform an elution.

14. The sample analyzer of claim 11, wherein a bottom of the accommodating hole in the elution zone is positioned higher than a bottom of the accommodating hole in the lysis zone, higher than a bottom of the accommodating hole in the washing zone, and higher than a bottom of the accommodating hole in the drying zone; and/or
the bottom of the accommodating hole in the lysis zone is positioned at the same height as the bottom of the accommodating hole in the washing zone and the bottom of the accommodating hole in the drying zone.

15. The sample analyzer of any one of claims 1 to 14, wherein the second functional assembly comprises a fourth support member, a heating member, and a mixing member, the fourth support member being configured to support the nucleic acid extraction container for performing an incubation, the heating member being configured to heat the liquid containing at least the sample and the reagent in the nucleic acid extraction container located on the fourth support member, and the mixing member being configured to mix the liquid containing at least the sample and the reagent in the nucleic acid extraction container located on the fourth support member.

16. The sample analyzer of claim 15, wherein the second functional assembly further comprises a first driving member configured to drive the fourth support member to move such that the fourth support member drives the nucleic acid extraction container to move; and
the mixing member comprises a plurality of magnets, wherein the plurality of magnets are alternately arranged on opposite sides of a movement trajectory of the nucleic acid extraction container in a horizontal direction.

17. The sample analyzer of claim 15, wherein the mixing member comprises at least a first magnet array and a second magnet array both extending horizontally, with each magnet array comprising a plurality of magnets spaced apart and mounted on a magnet bracket;
the fourth support member comprises a container receiving portion for placement of the nucleic acid extraction container;
the fourth support member and the mixing member are disposed with respect to each other such that when the nucleic acid extraction container is received in the container receiving portion, at least a bottom of the nucleic acid extraction container is located between the first magnet array and the second magnet array; and
the fourth support member and the mixing member are capable of moving horizontally relative to each other such that the mixing member is capable of mixing the liquid containing at least the sample and the magnetic beads in the nucleic acid extraction container received in the container receiving portion; and
the fourth support member and the mixing member are further disposed with respect to each other such that when the fourth support member and the mixing member move horizontally relative to each other, at least two magnets in the mixing member have different heights relative to the bottom of the nucleic acid extraction container received in the container receiving portion.

18. The sample analyzer of claim 17, wherein the at least two magnets in the mixing member have different heights relative to the fourth support member; or
the at least two magnets in the mixing member are mounted at different heights on the magnet bracket, such that the at least two magnets in the mixing member have different heights relative to the fourth support member.

19. The sample analyzer of claim 15, wherein the second functional assembly further comprises a third driving member, and the mixing member comprises a movable magnet bracket and a plurality of magnets fixed to the magnet bracket, wherein the third driving member is configured to drive the magnet bracket to move so as to drive the plurality of magnets fixed to the magnet bracket to move relative to the nucleic acid extraction container on the fourth support member, such that the magnetic beads in the nucleic acid extraction container placed on the fourth support member perform a three-dimensional movement in the liquid in the nucleic acid extraction container under the effect of the moving magnets; or
the second functional assembly further comprises a third driving member, the mixing member is configured to generate a magnetic field, and the third driving member is configured to drive relative movement between the fourth support member and the mixing member, such that the mixing member generates an alternately varying magnetic field relative to the nucleic acid extraction container on the fourth support member, enabling the magnetic beads in the nucleic acid extraction container to perform a three-dimensional movement in the liquid in the nucleic acid extraction container under the effect of the alternately varying magnetic field; or
the second functional assembly further comprises a third driving member, the fourth support member comprises at least a lysis zone, and the nucleic acid extraction container undergoes a lysis and capture stage in the lysis zone, wherein during the lysis and capture stage, cells in the sample in the nucleic acid extraction container are lysed to release nucleic acids, and the released nucleic acids are captured by the magnetic beads in the nucleic acid extraction container; the mixing member is configured to generate a magnetic field covering at least the lysis zone; and the third driving member is configured to drive relative movement between the fourth support member and the mixing member, such that the magnetic beads in the nucleic acid extraction container located in the lysis zone are moved so as to capture the released nucleic acids.

20. The sample analyzer of any one of claims 2 to 14, further comprising a frame assembly, wherein the nucleic acid extraction device is mounted on the frame assembly; and
the second functional assembly comprises a fourth support member and a first driving member, wherein the fourth support member is movably mounted on the frame assembly and configured to support the nucleic acid extraction container for performing an incubation, at least one first transfer station and a plurality of accommodating holes are formed on the fourth support member, with each accommodating hole being configured to accommodate one nucleic acid extraction container, and the first driving member is configured to drive the fourth support member to move relative to the frame assembly, such that the fourth support member drives each nucleic acid extraction container to move to the first transfer station; the first support member is fixed to the frame assembly; and the transfer assembly is configured to at least transfer the nucleic acid extraction container between the first transfer station and the first support member.

21. The sample analyzer of any one of claims 2 to 14, further comprising a frame assembly, wherein the nucleic acid extraction device is mounted on the frame assembly; and
the second functional assembly comprises a fourth support member, wherein the fourth support member is fixed to the frame assembly and configured to support the nucleic acid extraction container for performing an incubation, and at least one first transfer station and a plurality of accommodating holes are formed on the fourth support member, with each accommodating hole being configured to accommodate one nucleic acid extraction container; the first support member is fixed to the frame assembly; and the transfer assembly is configured to at least transfer the nucleic acid extraction container between the first transfer station and the first support member, and is configured to be capable of performing at least a three-dimensional movement.

22. The sample analyzer of any one of claims 1 to 14, further comprising:
a container providing device configured to provide the nucleic acid extraction container and the amplification reaction container;
a reagent storage compartment configured to store a first-type reagent and a second-type reagent, the first-type reagent being configured to react with the sample to obtain a nucleic acid extract, and the second-type reagent being configured to react with the nucleic acid extract for amplification;
a reagent dispensing device configured to dispense the first-type reagent stored in the reagent storage compartment into the nucleic acid extraction container, and to dispense the second-type reagent stored in the reagent storage compartment into the amplification reaction container; and
a conveying device configured to at least convey the nucleic acid extraction container provided by the container providing device to the nucleic acid extraction device, and to convey the amplification reaction container provided by the container providing device to the amplification device.

23. A sample analyzer, comprising:
a sample distribution device configured to aspirate a sample from a sample container and dispense at least part of the aspirated sample into a nucleic acid extraction container;
a nucleic acid extraction device configured to extract nucleic acids from a liquid containing at least the sample and a reagent in the nucleic acid extraction container to obtain a nucleic acid extract;
a pipetting device configured to transfer the nucleic acid extract from the nucleic acid extraction container to an amplification reaction container;
an amplification device configured to amplify the nucleic acid extract in the amplification reaction container to obtain a test solution; and
a test device configured to test the test solution,
wherein the nucleic acid extraction device comprises a first functional assembly, a second functional assembly, and a transfer assembly, the first functional assembly being disposed above or below the second functional assembly, the transfer assembly being configured to transfer the nucleic acid extraction container between the first functional assembly and the second functional assembly, the first functional assembly being configured to at least support the nucleic acid extraction container for performing a first functional operation, and the second functional assembly being configured to at least support the nucleic acid extraction container for performing a second functional operation, so as to perform nucleic acid extraction,
wherein the first functional operation and the second functional operation are either different functional operations or the same functional operation performed in sequence.

24. The sample analyzer of claim 23, wherein the first functional operation comprises at least one of a liquid aspiration operation, a liquid dispensing operation, and a magnetic attraction operation; and/or
the second functional operation comprises at least one of an incubation operation and a mixing operation.

25. The sample analyzer of claim 23 or 24, wherein the second functional assembly comprises a fourth support member and a mixing member, the fourth support member being configured to support the nucleic acid extraction container for performing an incubation operation, and the mixing member being configured to mix the liquid containing at least the sample and the reagent in the nucleic acid extraction container located on the fourth support member,
wherein the mixing member comprises at least a first magnet array and a second magnet array both extending horizontally, with each magnet array comprising a plurality of magnets spaced apart and mounted on a magnet bracket;
the fourth support member comprises a container receiving portion for placement of the nucleic acid extraction container;
the fourth support member and the mixing member are disposed with respect to each other such that when the nucleic acid extraction container is received in the container receiving portion, at least a bottom of the nucleic acid extraction container is located between the first magnet array and the second magnet array;
the fourth support member and the mixing member are capable of moving horizontally relative to each other such that the mixing member is capable of mixing the liquid containing at least the sample and magnetic beads in the nucleic acid extraction container received in the container receiving portion; and
the fourth support member and the mixing member are further disposed with respect to each other such that when the fourth support member and the mixing member move horizontally relative to each other, at least two magnets in the mixing member have different heights relative to the bottom of the nucleic acid extraction container received in the container receiving portion.

26. The sample analyzer of claim 23 or 24, wherein the second functional assembly comprises a fourth support member, a mixing member, and a third driving member, wherein the fourth support member is configured to support the nucleic acid extraction container for performing an incubation operation, the mixing member is configured to mix the liquid containing at least the sample and the reagent in the nucleic acid extraction container located on the fourth support member, the mixing member comprises a movable magnet bracket and a plurality of magnets fixed to the magnet bracket, and the third driving member is configured to drive the magnet bracket to move so as to drive the plurality of magnets fixed to the magnet bracket to move relative to the nucleic acid extraction container on the fourth support member, such that magnetic beads in the nucleic acid extraction container placed on the fourth support member perform a three-dimensional movement in the liquid in the nucleic acid extraction container under the effect of the moving magnets; or
the second functional assembly comprises a fourth support member, a mixing member, and a third driving member, wherein the fourth support member is configured to support the nucleic acid extraction container for performing an incubation operation, the mixing member is configured to generate a magnetic field to mix the liquid containing at least the sample and the reagent in the nucleic acid extraction container located on the fourth support member, and the third driving member is configured to drive relative movement between the fourth support member and the mixing member, such that the mixing member generates an alternately varying magnetic field relative to the nucleic acid extraction container on the fourth support member, enabling magnetic beads in the nucleic acid extraction container to perform a three-dimensional movement in the liquid in the nucleic acid extraction container under the effect of the alternately varying magnetic field; or
the second functional assembly comprises a fourth support member, a mixing member, and a third driving member, wherein the fourth support member is configured to support the nucleic acid extraction container for performing an incubation operation, the fourth support member comprises at least a lysis zone, and the nucleic acid extraction container undergoes a lysis and capture stage in the lysis zone, wherein during the lysis and capture stage, cells in the sample in the nucleic acid extraction container are lysed to release nucleic acids, and the released nucleic acids are captured by magnetic beads in the nucleic acid extraction container; the mixing member is configured to generate a magnetic field covering at least the lysis zone to mix at least the liquid containing at least the sample and the reagent in the nucleic acid extraction container located in the lysis zone; and the third driving member is configured to drive relative movement between the fourth support member and the mixing member, such that the magnetic beads in the nucleic acid extraction container located in the lysis zone are moved so as to capture the released nucleic acids.

27. A sample analyzer, comprising:
a sample distribution device configured to aspirate a sample from a sample container and dispense at least part of the aspirated sample into a nucleic acid extraction container;
a nucleic acid extraction device configured to extract nucleic acids from a liquid containing at least the sample and a reagent in the nucleic acid extraction container to obtain a nucleic acid extract;
a pipetting device configured to transfer the nucleic acid extract from the nucleic acid extraction container to an amplification reaction container;
an amplification device configured to amplify the nucleic acid extract in the amplification reaction container to obtain a test solution; and
a test device configured to test the test solution,
wherein the nucleic acid extraction device has at least two stations, with each station being configured to support the nucleic acid extraction container for performing a processing operation for nucleic acid extraction, at least two of the stations being vertically arranged one above the other, and the processing operation comprising at least one of an incubation operation, a mixing operation, a magnetic attraction operation, a liquid aspiration operation, and a liquid dispensing operation.

28. The sample analyzer of claim 27, wherein performing nucleic acid extraction on a liquid containing at least the sample and a reagent by the nucleic acid extraction device comprises: a process of performing lysis on the liquid containing at least the sample and the reagent and performing post-lysis processing.

29. The sample analyzer of claim 27 or 28, wherein the nucleic acid extraction device comprises a first-layer space and a second-layer space, the first-layer space being disposed above or below the second-layer space;
a liquid aspiration-dispensing station, a dedicated liquid aspiration station, and a dedicated liquid dispensing station are disposed in the first-layer space; and
a lysis incubation station, a cleaning incubation station, a drying incubation station, and an elution incubation station are disposed in the second-layer space,
wherein the lysis incubation station is configured to support the nucleic acid extraction container for performing a lysis incubation operation;
the cleaning incubation station is configured to support the nucleic acid extraction container for performing a cleaning incubation operation;
the drying incubation station is configured to support the nucleic acid extraction container for performing a drying incubation operation;
the elution incubation station is configured to support the nucleic acid extraction container for performing an elution incubation operation;
the liquid aspiration-dispensing station is configured to support the nucleic acid extraction container for performing a first liquid aspiration operation, and a first liquid dispensing operation for dispensing a cleaning liquid;
the dedicated liquid aspiration station is configured to support the nucleic acid extraction container for performing a second liquid aspiration operation; and
the dedicated liquid dispensing station is configured to support the nucleic acid extraction container for performing a second liquid dispensing operation for dispensing an elution reagent.

30. A sample analyzer, comprising:
a sample distribution device configured to aspirate a sample from a sample container and dispense at least part of the aspirated sample into a nucleic acid extraction container;
a nucleic acid extraction device configured to perform a nucleic acid extraction operation on a liquid containing at least the sample and a reagent in the nucleic acid extraction container to obtain a nucleic acid extract;
a pipetting device configured to transfer the nucleic acid extract from the nucleic acid extraction container to an amplification reaction container;
an amplification device configured to amplify the nucleic acid extract in the amplification reaction container to obtain a test solution; and
a test device configured to test the test solution,
wherein the nucleic acid extraction device comprises a lysis module, a cleaning module, an elution module, and a transfer assembly, at least two of the lysis module, the cleaning module and the elution module being arranged in a vertical direction, the lysis module being configured to support the nucleic acid extraction container for performing a lysis operation, the cleaning module being configured to support the nucleic acid extraction container for performing a cleaning operation, and the elution module being configured to support the nucleic acid extraction container for performing an elution operation; and the transfer assembly is configured to sequentially transfer the nucleic acid extraction container to the lysis module, the cleaning module, and the elution module.

31. The sample analyzer of claim 30, wherein performing nucleic acid extraction on a liquid containing at least the sample and a reagent by the nucleic acid extraction device comprises: a process of performing lysis on the liquid containing at least the sample and the reagent and performing post-lysis processing.

32. The sample analyzer of claim 30 or 31, wherein the nucleic acid extraction device further comprises a drying module disposed below or above at least one of the lysis module, the cleaning module and the elution module, the drying module being configured to support the nucleic acid extraction container for performing a drying operation, and the transfer assembly is configured to sequentially transfer the nucleic acid extraction container to the lysis module, the cleaning module, the drying module, and the elution module.
